# EUROPEAN PATENT APPLICATION

(11) **EP 2 033 640 A2**
(43) Date of publication of application: **11.03.2009**
(21) Application number: 08164583.0
(22) Date of filing: 18.02.2005
(51) Int. Cl.: A61K 31/352, A61P 35/00, A61P 35/04

(54) **Beta-lapachone for the treatment of lung cancer**

(30) Priority: 20.02.2004 US 545915 P
(62) Divisional of application: 05723506.1
(71) Applicant: ARQULE, INC., Woburn, MA 01801-5140 (US)
(72) Inventor: Li, Chiang J., West Roxbury, MA 02132 (US); Li, YouZhi, Westwood, MA 02090 (US)
(74) Representative: Crump, Julian Richard John

(57) **Abstract**

A composition comprising β-lapachone, or a pharmaceutically acceptable salt thereof, in combination with a pharmaceutically acceptable carrier, for use in the treatment or prevention of a cell proliferative disorder of the lung. Said cell proliferative disorder may be lung cancer.

## Description

### BACKGROUND OF THE INVENTION

Lung cancer is the leading cause of cancer mortality in the United States ("Cancer Facts and Figures 2003," American Cancer Society). Lung cancer is particularly insidious because symptoms of early-stage, localized disease are nonspecific and are frequently attributed to the effects of smoking. By the time the patient seeks medical attention, the disease is usually advanced so that complete surgical resection is possible in less than 30% of all cases, and the overall 5-year survival rate in less than 15%. ("Cancer of the Lung: Cancer Screening and Early Detection," in Cancer Medicine, 5th Edition, Bast et al. eds., B.C. Decker Inc., Hamilton, Ontario).

Surgery and radiotherapy may be curative if a cancer is found early, but current drug therapies for metastatic disease are mostly palliative and seldom offer a long-term cure. Even with the new chemotherapies entering the market, improvement in patient survival is measured in months rather than in years, and the need continues for new drugs effective both in combination with existing agents as first line therapy and as second and third line therapies in treatment of resistant tumors.

Cancer cells are by definition heterogeneous. For example, within a single tissue or cell type, multiple mutational 'mechanisms' may lead to the development of cancer. As such, heterogeneity frequently exists between cancer cells taken from tumors of the same tissue and same type that have originated in different individuals. Frequently-observed mutational 'mechanisms' associated with some cancers may differ between one tissue type and another (e.g., frequently-observed mutational 'mechanisms' leading to colon cancer may differ from frequently-observed 'mechanisms' leading to leukemias). It is therefore often difficult to predict whether a particular cancer will respond to a particular chemotherapeutic agent. (Cancer Medicine, 5th Edition, Bast et al, eds., B.C. Decker Inc., Hamilton, Ontario).

β-lapachone is an agent with a reported anti-cancer activity in a limited number of non-lung cancers. For example, there is reported a method and composition for the treatment of tumors, which comprises the administration of an effective amount of β-lapachone, in combination with a taxane derivative (WO00/61142). Additionally, U.S. Pat. No. 6,245,807 discloses the use of β-lapachone, amongst other β-lapachone derivatives, for use in treatment of human prostate disease. As a single agent, β-lapachone has also been reported to decrease the number of tumors, reduce tumor size, or increase survival time, or a combination of these in xenotransplant mouse models of human ovarian cancer (Li, C.J. et al., (1999) Proc. Natl. Acad. Sci. USA, 96(23): 13369-13374), human prostate cancer (Li, C.J. et al., (1999) Proc. Natl. Acad. Sci. USA, 96(23): 13369-13374), human breast cancer (Li, C.J. et al., (2000) AACR Proc., p. 9), and human multiple myeloma (WO 03/011224).

While β-lapachone, alone or in combination with other agents, has been reported to reduce tumor size in a limited number of tumor models it has not been reported to be an effective agent for the treatment of human lung cancers.

### SUMMARY OF THE INVENTION

The present invention provides a method of treating lung cancer, comprising administering to a subject in need thereof a therapeutically effective amount of β-lapachone, or a pharmaceutically acceptable salt thereof, in combination with a pharmaceutically acceptable carrier, where said lung cancer is treated.

The present invention also provides a method of treating metastatic lung cancer comprising administering to a subject in need thereof a therapeutically effective amount of β-lapachone, or a pharmaceutically acceptable salt thereof, in combination with a pharmaceutically acceptable carrier, where said metastatic lung cancer is treated.

The present invention also provides a method of treating or preventing a cell proliferative disorder of the lung, comprising administering to a subject in need thereof a therapeutically effective amount of β-lapachone, or a pharmaceutically acceptable salt thereof, in combination with a pharmaceutically acceptable carrier, where said cell proliferative disorder of the lung is treated or prevented.

The present invention also provides a method for inducing cell death in a lung cancer cell, comprising contacting said lung cancer cell with an effective amount of β-lapachone, or a pharmaceutically acceptable salt thereof, where said contacting induces said cell death in said lung cancer cell.

The present invention provides a method of treating lung cancer, comprising administering to a subject in need thereof a therapeutically effective amount of β-lapachone, or a pharmaceutically acceptable salt, prodrug, metabolite, analog or derivative thereof, in combination with a pharmaceutically acceptable carrier, where said lung cancer is treated.

The present invention also provides a method of treating metastatic lung cancer comprising administering to a subject in need thereof a therapeutically effective amount of β-lapachone, or a pharmaceutically acceptable salt, prodrug, metabolite, analog or derivative thereof, in combination with a pharmaceutically acceptable carrier, where said metastatic lung cancer is treated.

The present invention also provides a method of treating or preventing a cell proliferative disorder of the lung, comprising administering to a subject in need thereof a therapeutically effective amount of β-lapachone, or a pharmaceutically acceptable salt, prodrug, metabolite, analog or derivative thereof, in combination with a pharmaceutically acceptable carrier, where said cell proliferative disorder of the lung is treated or prevented.

The present invention also provides a method for inducing cell death in a lung cancer cell, comprising contacting said lung cancer cell with an effective amount of β-lapachone, or a pharmaceutically acceptable salt, prodrug, metabolite, analog or derivative thereof, where said contacting induces said cell death in said lung cancer cell.

The present invention also provides a method of treating lung cancer, comprising administering to a subject in need thereof a therapeutically effective amount of a) β-lapachone, or a pharmaceutically acceptable salt thereof, in combination with a pharmaceutically acceptable carrier, and b) gemcitabine, or a pharmaceutically acceptable salt thereof, in combination with a pharmaceutically acceptable carrier, where the lung cancer is treated.

The preset invention also provides a method of treating metastatic lung cancer, comprising administering to a subject in need thereof a therapeutically effective amount of a) β-lapachone, or a pharmaceutically acceptable salt thereof, in combination with a pharmaceutically acceptable carrier, and b) gemcitabine, or a pharmaceutically acceptable salt thereof, in combination with a pharmaceutically acceptable carrier, where the metastatic lung cancer is treated.

The present invention also provides a method of treating a cell proliferative disorder of the lung, comprising administering to a subject in need thereof a therapeutically effective amount of a) β-lapachone, or a pharmaceutically acceptable salt thereof, in combination with a pharmaceutically acceptable carrier, and b) gemcitabine, or a pharmaceutically acceptable salt thereof, in combination with a pharmaceutically acceptable carrier, where the cell proliferative disorder of the lung is treated.

The present invention also provides a method for inducing cell death in a lung cancer cell, comprising contacting the lung cancer cell with an effective amount of a) β-lapachone, or a pharmaceutically acceptable salt thereof, and b) gemcitabine, or a pharmaceutically acceptable salt thereof, where the contacting induces the cell death in the lung cancer cell.

The present invention also provides a method of treating lung cancer, comprising administering to a subject in need thereof a therapeutically effective amount of a) β-lapachone, or a pharmaceutically acceptable salt, prodrug, metabolite, analog or derivative thereof, in combination with a pharmaceutically acceptable carrier, and b) gemcitabine, or a pharmaceutically acceptable salt, prodrug, metabolite, analog or derivative thereof, in combination with a pharmaceutically acceptable carrier, where the lung cancer is treated.

The present invention also provides a method of treating metastatic lung cancer, comprising administering to a subject in need thereof a therapeutically effective amount of a) β-lapachone, or a pharmaceutically acceptable salt, prodrug, metabolite, analog or derivative thereof, in combination with a pharmaceutically acceptable carrier, and b) gemcitabine, or a pharmaceutically acceptable salt, prodrug, metabolite, analog or derivative thereof, in combination with a pharmaceutically acceptable carrier, where the metastatic lung cancer is treated.

The present invention also provides a method of treating a cell proliferative disorder of the lung, comprising administering to a subject in need thereof a therapeutically effective amount of a) β-lapachone, or a pharmaceutically acceptable salt, prodrug, metabolite, analog or derivative thereof, in combination with a pharmaceutically acceptable carrier, and b) gemcitabine, or a pharmaceutically acceptable salt, prodrug, metabolite, analog or derivative thereof, in combination with a pharmaceutically acceptable carrier, where the cell proliferative disorder of the lung is treated.

The present invention also provides a method for inducing cell death in a lung cancer cell, comprising contacting the lung cancer cell with an effective amount of a) β-lapachone, or a pharmaceutically acceptable salt, prodrug, metabolite, analog or derivative thereof, and b) gemcitabine, or a pharmaceutically acceptable salt, prodrug, metabolite, analog or derivative thereof, where the contacting induces the cell death in the lung cancer cell.

The present invention provides a method of treating lung cancer, comprising administering to a subject in need thereof a therapeutically effective amount of β-lapachone or pharmaceutically acceptable salt thereof, or a metabolite thereof, in combination with a pharmaceutically acceptable carrier, where the β-lapachone or pharmaceutically acceptable salt thereof, or a metabolite thereof, treats the lung cancer.

The present invention also provides a method of treating lung cancer, comprising administering to a subject in need thereof a therapeutically effective amount of β-lapachone or pharmaceutically acceptable salt thereof, or a metabolite thereof, in combination with a pharmaceutically acceptable carrier, and activating one or more cell cycle checkpoints in one or more lung cancer cells, where the β-lapachone or pharmaceutically acceptable salt thereof, or a metabolite thereof, treats the lung cancer.

The present invention further provides a method of treating lung cancer, comprising administering to a subj ect in need thereof a therapeutically effective amount of β-lapachone or pharmaceutically acceptable salt thereof, or a metabolite thereof, in combination with a pharmaceutically acceptable carrier, and activating one or more cell cycle checkpoint pathways in one or more lung cancer cells, where the β-lapachone or pharmaceutically acceptable salt thereof, or a metabolite thereof, treats the lung cancer.

The present invention also provides a method of treating lung cancer, comprising administering to a subject in need thereof a therapeutically effective amount of β-lapachone or pharmaceutically acceptable salt thereof, or a metabolite thereof, in combination with a pharmaceutically acceptable carrier, and activating one or more cell cycle checkpoint regulators in one or more lung cancer cells, where the β-lapachone or pharmaceutically acceptable salt thereof, or a metabolite thereof, treats the lung cancer.

The present invention further provides a method of treating lung cancer, comprising administering to a subject in need thereof a therapeutically effective amount of β-lapachone or pharmaceutically acceptable salt thereof, or a metabolite thereof, in combination with a pharmaceutically acceptable carrier, and activating cell death selectively in one or more lung cancer cells, where the β-lapachone or pharmaceutically acceptable salt thereof, or a metabolite thereof, treats the lung cancer.

The present invention also provides a method of treating or preventing a cell proliferative disorder of the lung, comprising administering to a subject in need thereof a therapeutically effective amount of β-lapachone or pharmaceutically acceptable salt thereof, or a metabolite thereof, in combination with a pharmaceutically acceptable carrier, where the β-lapachone or pharmaceutically acceptable salt thereof, or a metabolite thereof, treats or prevents the cell proliferative disorder of the lung.

The present invention provides a method of treating lung cancer, comprising administering to a subject in need thereof a therapeutically effective amount of an analog or derivative of β-lapachone or pharmaceutically acceptable salt thereof, or a metabolite thereof, in combination with a pharmaceutically acceptable carrier, where the analog or derivative of β-lapachone or pharmaceutically acceptable salt thereof, or a metabolite thereof, treats the lung cancer.

The present invention also provides a method of treating lung cancer, comprising administering to a subject in need thereof a therapeutically effective amount of an analog or derivative of β-lapachone or pharmaceutically acceptable salt thereof, or a metabolite thereof, in combination with a pharmaceutically acceptable carrier, and activating one or more cell cycle checkpoints in one or more lung cancer cells, where the analog or derivative of β-lapachone or pharmaceutically acceptable salt thereof, or a metabolite thereof, treats the lung cancer.

The present invention further provides a method of treating lung cancer, comprising administering to a subject in need thereof a therapeutically effective amount of an analog or derivative β-lapachone or pharmaceutically acceptable salt thereof, or a metabolite thereof, in combination with a pharmaceutically acceptable carrier, and activating one or more cell cycle checkpoint pathways in one or more lung cancer cells, where the β-lapachone or pharmaceutically acceptable salt thereof, or a metabolite thereof, treats the lung cancer.

The present invention also provides a method of treating lung cancer, comprising administering to a subject in need thereof a therapeutically effective amount of an analog or derivative of β-lapachone or pharmaceutically acceptable salt thereof, or a metabolite thereof, in combination with a pharmaceutically acceptable carrier, and activating one or more cell cycle checkpoint regulators in one or more lung cancer cells, where the analog or derivative of β-lapachone or pharmaceutically acceptable salt thereof, or a metabolite thereof, treats the lung cancer.

The present invention further provides a method of treating lung cancer, comprising administering to a subject in need thereof a therapeutically effective amount of an analog or derivative of β-lapachone or pharmaceutically acceptable salt thereof, or a metabolite thereof, in combination with a pharmaceutically acceptable carrier, and activating cell death selectively in one or more lung cancer cells, where the analog or derivative of β-lapachone or pharmaceutically acceptable salt thereof, or a metabolite thereof, treats the lung cancer.

The present invention also provides a method of treating or preventing a cell proliferative disorder of the lung, comprising administering to a subject in need thereof a therapeutically effective amount of an analog or derivative of β-lapachone or pharmaceutically acceptable salt thereof, or a metabolite thereof, in combination with a pharmaceutically acceptable carrier, where the analog or derivative of β-lapachone or pharmaceutically acceptable salt thereof, or a metabolite thereof, treats or prevents the cell proliferative disorder of the lung.

The present invention also provides a method of treating or preventing a cell proliferative disorder of the lung, comprising administering to a subject in need thereof a therapeutically effective amount of β-lapachone or pharmaceutically acceptable salts thereof, or a metabolite thereof, in combination with a pharmaceutically acceptable carrier, and activating one or more cell cycle checkpoints in a lung cell, where the β-lapachone or pharmaceutically acceptable salts thereof, or a metabolite thereof, treats or prevents a cell proliferative disorder of the lung.

The present invention also provides a method of treating or preventing a cell proliferative disorder of the lung, comprising administering to a subject in need thereof a therapeutically effective amount of β-lapachone or pharmaceutically acceptable salts thereof, or a metabolite thereof, in combination with a pharmaceutically acceptable carrier, and activating one or more cell cycle checkpoint pathways in a lung cell, where the β-lapachone or pharmaceutically acceptable salts thereof, or a metabolite thereof, treats or prevents a cell proliferative disorder of the lung.

The present invention also provides a method of treating or preventing a cell proliferative disorder of the lung, comprising administering to a subject in need thereof a therapeutically effective amount of β-lapachone or pharmaceutically acceptable salts thereof, or a metabolite thereof, in combination with a pharmaceutically acceptable carrier, and activating one or more cell cycle checkpoint regulators in a lung cell, where the β-lapachone or pharmaceutically acceptable salts thereof, or a metabolite thereof, treats or prevents a cell proliferative disorder of the lung.

The present invention also provides a method of treating or preventing a cell proliferative disorder of the lung, comprising administering to a subject in need thereof a therapeutically effective amount of β-lapachone or pharmaceutically acceptable salts thereof, or a metabolite thereof, in combination with a pharmaceutically acceptable carrier, and activating cell death selectively in lung cell, where the β-lapachone or pharmaceutically acceptable salts thereof, or a metabolite thereof, treats or prevents a cell proliferative disorder of the lung.

### BRIEF DESCRIPTION OF THE FIGURES

Figure 1 sets forth a schematic of the proposed points of action of β-lapachone and Taxol on the cell cycle.
Figure 2 sets forth an effect of β-Lapachone on survival of human lung cancer cell lines in the NCI60 assay *in vitro.*
Figure 3 sets forth an effect of β-Lapachone on the growth of xenografted A549 human lung tumors in an athymic nude mouse model.
Figure 4 sets forth an effect of β-Lapachone, administered in monotherapy or in combination with gemcitabine (GEMZAR®), on the growth of xenografted A549 human lung tumors in an athymic nude mouse model.

### DETAILED DESCRIPTION OF THE INVENTION

The present invention provides a method of treating lung cancer, including metastatic lung cancer, comprising administering to a subject in need thereof a therapeutically effective amount of β-lapachone, or a pharmaceutically acceptable salt, prodrug, metabolite, analog or derivative thereof, in combination with a pharmaceutically acceptable carrier, where the lung cancer is treated. The present invention also provides a method of treating or preventing a cell proliferative disorder of the lung, comprising administering to a subject in need thereof a therapeutically effective amount of β-lapachone, or a pharmaceutically acceptable salt, prodrug, metabolite, analog or derivative thereof, in combination with a pharmaceutically acceptable carrier, where the cell proliferative disorder of the lung is treated or prevented. The invention also provides the use of β-lapachone for the preparation of a medicament useful for the treatment of lung cancer. The invention also provides the use of β-lapachone for the preparation of a medicament useful for the treatment or prevention of a cell proliferative disorder of the lung. The invention also provides the use of an analog or derivative of β-lapachone for the preparation of a medicament useful for the treatment of lung cancer. The invention also provides the use of an analog or derivative of β-lapachone for the preparation of a medicament useful for the treatment or prevention of a cell proliferative disorder of the lung.

While not limited by theory, the present invention includes and is based in part on an understanding of, and methods for, the activation of cell cycle checkpoints by modulators of cell cycle checkpoint activation (e.g., β-lapachone, or a pharmaceutically acceptable salt, prodrug, metabolite, analog or derivative thereof). The activation of cell cycle checkpoints in general is referred to as Activated Checkpoint Therapy™, or ACT™. Briefly, many cancer cells are defective in their cell cycle checkpoint functions secondary to mutations in one of their molecular modulators, e.g., p53. It is in part, for this reason, that cancer cells have accumulated genetic errors during the carcinogenic process. Therapeutic agents that activate cell cycle checkpoint functions can selectively promote cell death in cancer cells, since cell death appears to be induced at least in part by the conflict between the uncontrolled-proliferation drive in cancer cells and the checkpoint delays induced artificially. ACT™ takes advantage of the tendency of cell death to occur at checkpoints during the cell proliferation cycle by activating one or more checkpoints, thereby producing conflicting signals regarding cell cycle progression versus arrest. If more than one checkpoint is activated, cancer cells with uncontrolled proliferation signals and genetic abnormalities are blocked at multiple checkpoints, creating "collisions" that promote synergistic cell death.

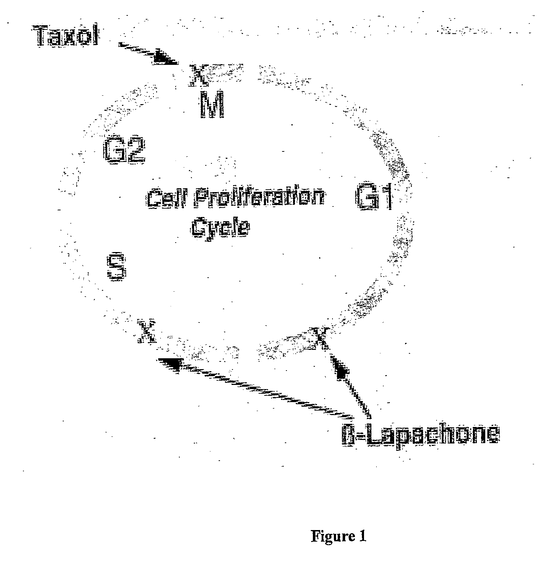

ACT™ offers selectivity against cancer cells as compared to normal cells and is therefore safer than less selective therapies. First, the ACT™ method activates but does not disrupt cell cycle checkpoints. Second, normal cells with well-controlled proliferation signals can be delayed at checkpoints in a regulated fashion, resulting in no cell death-prone collisions. Third, normal cells with intact G1 checkpoint control are expected to arrest in G1. Cancer cells, on the other hand, are expected to be delayed in S-, G2-, and M-phases, since most cancer cells harbor G1 checkpoint defects, making cancer cells more sensitive to drugs imposing S and M phase checkpoints. β-lapachone is a G1 and S phase compound, and contacting a cell with β-lapachone results in activation of a G1 or S cell cycle checkpoint. Figure 1 sets forth a schematic of the proposed points of action of β-lapachone and Taxol on the cell cycle.

The term "modulator of cell cycle checkpoint activation," as used herein, refers to a compound capable of altering checkpoint activation in cells (in preferred embodiments, activating one or more cell cycle checkpoints), preferably by activating checkpoint-mediated DNA repair mechanisms, or by reinstating checkpoint activity that has been lost due to a malfunction or mutation in the cellular pathways that regulate cell cycle activity. As is known in the art, major cell cycle checkpoints occur at G₁/S phase and at the G₂/M phase transitions. In a model, four major cell cycle checkpoints monitor the integrity of genetic material. DNA synthesis begins only past the restriction point (R point), where the cell determines if preparation during G1 has been satisfactory for cell cycle continuation. The second checkpoint occurs during replication initiation in S phase. The third and fourth checkpoints take place in G2 phase and M phase, respectively. Modulation of cell cycle checkpoint activation is further discussed in, *e.g.,* C.J. Li et al. Proc. Natl. Acad. Sci. USA (1999), 96(23), 13369-13374, and Y. Li et al. Proc. Natl. Acad. Sci. USA (2003), 100(5), 2674-2678, and PCT Publication WO 00/61142 (Pardee *et αl*.). Preferred modulators of cell cycle checkpoint activation for use in the present invention induce checkpoint activation (*i.e.,* activate one or more cell cycle checkpoints, preferably at G₁/S phase), preferably without causing substantial DNA damage. In addition, certain preferred modulators of cell cycle checkpoint activation are capable of increasing the level or activity of E2F (more preferably E2F1) in a cell. Methods for screening for modulators of cell cycle checkpoint activation, including compounds capable of elevating E2F activity or levels in a cell, include those that are disclosed in PCT Patent Application No. PCT/US03/22631 to Li et al. In certain embodiments, preferred modulators of cell cycle checkpoint activation are capable of increasing the level or activity of E2F in a cell by an amount sufficient to cause cell death (e.g., apoptosis) if the cell is a cancerous cell. More preferred modulators of cell cycle checkpoint activation are capable of raising the level or activity of E2F1 in a cell by an amount sufficient to cause cell death (e.g., apoptosis) if the cell is a cancerous cell. In one aspect, a modulator of cell cycle checkpoint activation is not β-lapachone.

Again not limited by theory, cellular response to DNA damage is regulated by the ATM/ATR signal transduction pathway, in which ATM and ATR are protein kinases of the phosphatidyl-inositol-3 kinase family (PI3K). In response to DNA damage, ATM and ATR phosphorylate Chk2 and Chk1 respectively, which in turn activate a variety of substrates involved in arresting cells at the G1/S phase of the cell cycle, as well as inducing and activating proteins involved in DNA repair. Chk2 has been shown to activate proteins involved in DNA repair including the tumor suppressor BRCA1, thereby enhancing DNA repair capacity following DNA damage. Chk2 has also been shown to stabilize p53 both by directly phosphorylating p53, and by inhibiting Mdm2, a ubiquitin ligase that targets p53 for degradation. Under such conditions, increased levels of p53 lead to G1/S arrest, DNA repair, and apoptosis in cells with irreparable DNA damage. Again not limited by theory, it is believed that Chk2 is an important cell cycle regulator, which, depending on the conditions, can induce cell cycle arrest and DNA repair, or initiate cell death (e.g., apoptosis) if DNA damage is too severe. In certain embodiments, preferred modulators of cell cycle checkpoint activation are capable of increasing the level or activity of Chk2 in a cell by an amount sufficient to cause cell death (e.g., apoptosis) if the cell is a cancerous cell.

Again not limited by theory, E2F1 is one of related proteins in the E2F family of nuclear transcription factors, which family is critically important in regulation of the cell cycle. E2F1 is required for cellular proliferation by promoting passage through the G1/S checkpoint. During proliferation of normal cells, transcriptionally active E2F1 is liberated from an inactive E2F1/Rb complex following phosphorylation of Rb. E2F1 levels rise, promoting progression through G1. As the cell moves toward the end of S phase, E2F1 levels must decline for progress to continue. Sustained elevation of E2F1 at this point in the cell cycle causes activation of the S phase checkpoint, and subsequent cell death (e.g., by apoptosis). Thus, depending on the phase of the cell cycle and dynamics of E2F1 elevation, this regulatory protein may either promote cellular proliferation, induce cell cycle delay, DNA repair or cell death. During the G1 phase of the cell cycle, phosphorylation of Rb results in dissociation of Rb-E2F1 complexes, liberating active E2F1, which then stimulates entry into S phase by promoting transcription of key cell cycle effectors. During S-phase, E2F1 must be degraded for progress to continue. In the presence of DNA damage, however, E2F1 levels increase rather than decrease, causing cell cycle delay, DNA repair, and, if damage is severe, cell death. As used herein, "E2F" is the E2F transcription factor family (including but not limited to E2F-1, E2F-2, E2F-3).

As used herein, "a cell cycle checkpoint pathway" refers to a biochemical pathway that is involved in modulation of a cell cycle checkpoint. A cell cycle checkpoint pathway may have stimulatory or inhibitory effects, or both, on one or more functions comprising a cell cycle checkpoint. A cell cycle checkpoint pathway is comprised of at least two compositions of matter, preferably proteins, both of which contribute to modulation of a cell cycle checkpoint. A cell cycle checkpoint pathway may be activated through an activation of one or more members of the cell cycle checkpoint pathway. Preferably, a cell cycle checkpoint pathway is a biochemical signaling pathway.

As used herein, "cell cycle checkpoint regulator" refers to a composition of matter that can function, at least in part, in modulation of a cell cycle checkpoint. A cell cycle checkpoint regulator may have stimulatory or inhibitory effects, or both, on one or more functions comprising a cell cycle checkpoint. In one aspect, a cell cycle checkpoint regulator is a protein. In another aspect, a cell cycle checkpoint regulator is a not a protein. In one aspect, a cell cycle checkpoint regulator is selected from the group consisting of ATM, ATR, Chk1, Chk2, E2F1, BRCA1, Rb, p53, p21, Mdm2, Cdc2, Cdc25, and 14-4-3[sigma].

### I. Compositions

As used herein, the phrase "β-lapachone" refers to 3,4-dihydro-2,2-dimethyl-2H-naphtho[1,2-b]pyran-5,6-dione and derivatives and analogs thereof, and has the chemical structure:

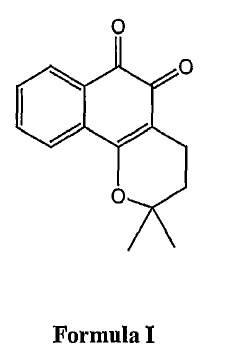

Preferred derivatives and analogs are discussed below.
β -Lapachone (3,4-dihydro-2,2-dimethyl-2H-naphtho [1,2-b] pyran-5, 6-dione), a simple non-water soluble orthonapthoquinone, was first isolated in 1882 by Paterno from the heartwood of the lapacho tree (*See* Hooker, SC, (1936) I.Am. Chem. Soc. 58:1181-1190; Goncalves de Lima, O, et al., (1962) Rev. Inst. Antibiot. Univ. Recife. 4:3-17). The structure of β-Lapachone was established by Hooker in 1896 and it was first synthesized by Fieser in 1927 (Hooker, SC, (1936) I. Am. Chem. Soc. 58:1181-1190). β-Lapachone can, for example, be obtained by simple sulfuric acid treatment of the naturally occurring lapachol, which is readily isolated from *Tabebuia avellenedae* growing mainly in Brazil, or is easily synthesized from seeds of lomatia growing in Australia (Li, CJ, et al., (1993) J. Biol. Chem. 268:22463-33464). Methods for formulating β-Lapachone or its derivatives or analogs can be accomplished as described in U.S. Patent No. 6,458,974 and U.S. Publication No. US-2003-0091639-A1.

As used herein, derivatives or analogs of β-Lapachone include, for example, 3,4-dihydro-2,2-dimethyl-3-(3-methyl-2-butenyl)-2H-naphtho[1,2-b]pyran-5,6-dione, 3,4-dihydro-2,2-dimethyl-2H-naphtho[1,2-b]thiopyran-5,6-dione and 3,4-dihydro-4,4-dimethyl-2H-naphtho[1,2-b]thiopyran-5,6-dione. Other derivatives or analogs of β-lapachone are described in PCT International Application PCT/US93/07878 (WO94/04145), and U.S. Pat. No. 6,245,807. PCT International Application PCT/LTS00/10169 (WO 00/61142), discloses β-lapachone, which may have a variety of substituents at the 3- position as well as in place of the methyl groups attached at the 2-position. U.S. Patent Nos. 5,763,625, 5,824,700, and 5,969,163, disclose analogs and derivatives with a variety of substituents at the 2-, 3- and 4-positions. Furthermore, a number of journals report β-lapachone analogs and derivatives with substituents at one or more of the following positions: 2-, 3-, 8- and/or 9-positions, (See, Sabba et al., (1984) J Med Chem 27:990-994 (substituents at the 2-, 8- and 9- positions); (Portela and Stoppani, (1996) Biochem Pharm 51:275-283 (substituents at the 2- and 9- positions); Goncalves et al., (1998) Molecular and Biochemical Parasitology 1:167-176 (substituents at the 2- and 3- positions)). Other derivatives or analogs of β-lapachone have sulfur-containing hetero-rings in the "α" and "β" positions of lapachone (Kurokawa S, (1970) Bulletin of The Chemical Society of Japan 43:1454-1459; Tapia, RA et al., (2000) Heterocycles 53(3):585-598; Tapia, RA et al., (1997) Tetrahedron Letters 38(1):153-154; Chuang, CP et al., (1996) Heterocycles 40(10):2215-2221; Suginome H et al., (1993) Journal of the Chemical Society, Chemical Communications 9:807-809; Tonholo J et al., (1988) Journal of the Brazilian Chemical Society 9(2):163-169; and Krapcho AP et al., (1990) Journal of Medicinal Chemistry 33(9):2651-2655).

Further, derivatives or analogs of β**-**lapachone include reduced β-lapachone (e.g., Formula 1a, in which R' and R" are both hydrogen) and derivatives of reduced β-lapachone (see, e.g., Formula 1a, in which R' and R" are each independently hydrogen, C₁-C₆ alkyl, C₁-C₆ alkylcarbonyl, or a pharmaceutically acceptable salt).

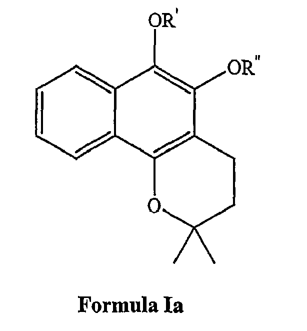

While β-lapachone is the preferred G1/S-phase compound for use in the composition in accordance with the present invention, the invention is not limited in this respect, and β-lapachone derivatives or analogs, such as lapachol, and pharmaceutical compositions and formulations thereof are part of the present invention. Such β-lapachone analogs include those recited in PCT International Application PCT/US93/07878 (WO 94/04145), which discloses compounds of the formula: 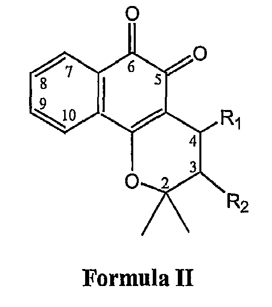 where R and R₁ are each independently hydrogen, substituted and unsubstituted aryl, substituted and unsubstituted alkenyl, substituted and unsubstituted alkyl and substituted or unsubstituted alkoxy. The alkyl groups preferably have from 1 to about 15 carbon atoms, more preferably from 1 to about 10 carbon atoms, still more preferably from 1 to about 6 carbon atoms. The term alkyl unless otherwise modified refers to both cyclic and noncyclic groups, although of course cyclic groups will comprise at least three carbon ring members. Straight or branched chain noncyclic alkyl groups are generally more preferred than cyclic groups. Straight chain alkyl groups are generally more preferred than branched. The alkenyl groups preferably have from 2 to about 15 carbon atoms, more preferably from 2 to about 10 carbon atoms, still more preferably from 2 to 6 carbon atoms. Especially preferred alkenyl groups have 3 carbon atoms (i.e., 1-propenyl or 2-propenyl), with the allyl moiety being particularly preferred. Phenyl and napthyl are generally preferred aryl groups. Alkoxy groups include those alkoxy groups having one or more oxygen linkage and preferably have from 1 to 15 carbon atoms, more preferably from 1 to about 6 carbon atoms. The substituted R and R₁ groups may be substituted at one or more available positions by one or more suitable groups such as, for example, alkyl groups such as alkyl groups having from 1 to 10 carbon atoms or from 1 to 6 carbon atoms, alkenyl groups such as alkenyl groups having from 2 to 10 carbon atoms or 2 to 6 carbon atoms, aryl groups having from six to ten carbon atoms, halogen such as fluoro, chloro and bromo, and N, O and S, including heteroalkyl, *e.g.,* heteroalkyl having one or more hetero atom linkages (and thus including alkoxy, aminoalkyl and thioalkyl) and from 1 to 10 carbon atoms or from 1 to 6 carbon atoms.

Other β-lapachone analogs contemplated in accordance with the present invention include those described in U.S. Patent No. 6,245,807, which discloses β-lapachone analogs and derivatives having the structure: 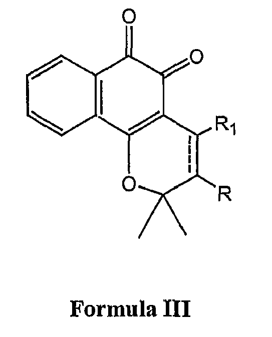 where R and R₁ are each independently selected from hydrogen, hydroxy, sulfhydryl, halogen, substituted alkyl, unsubstituted alkyl, substituted alkenyl, unsubstituted alkenyl, substituted aryl, unsubstituted aryl, substituted alkoxy, unsubstituted alkoxy, and salts thereof, where the dotted double bond between the ring carbons represents an optional ring double bond.

Additional β-lapachone analogs and derivatives are recited in PCT International Application PCT/US00/10169 (WO00/61142), which disclose compounds of the structure: 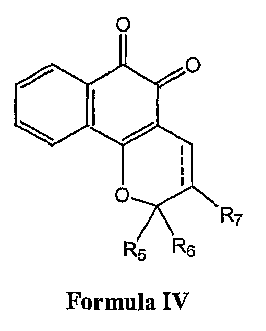 where R₅ and R₆ may be independently selected from hydroxy, C₁-C₆ alkyl, C₁-C₆ alkenyl, C₁-C₆ alkoxy, C₁-C₆ alkoxycarbonyl, --(CH₂)ₙ-phenyl; and R₇ is hydrogen, hydroxyl, C₁-C₆ alkyl, C₁-C₆ alkenyl, C₁-C₆ alkoxy, C₁-C₆ alkoxycarbonyl, --(CH₂)ₙ-amino, --(CH₂)ₙ-aryl, --(CH₂)ₙ-heteroaryl, --(CH₂)ₙ-heterocycle, or --(CH₂)ₙ-phenyl, wherein n is an integer from 0 to 10.

Other β-lapachone analogs and derivatives are disclosed in U.S. Pat. No. 5,763,625, U.S. Pat. No. 5,824,700, and U.S. Pat. No. 5,969,163, as well is in scientific journal articles, such as Sabba et al., J Med Chem 27:990-994 (1984), which discloses β-lapachone with substitutions at one or more of the following positions: 2-, 8- and/or 9- positions. *See also* Portela et al., Biochem Pharm 51:275-283 (1996) (substituents at the 2- and 9- positions); Maruyama et al., Chem Lett 847-850 (1977); Sun et al., Tetrahedron Lett 39:8221-8224 (1998); Goncalves et al., Molecular and Biochemical Parasitology 1:167-176 (1998) (substituents at the 2- and 3-positions); Gupta et al., Indian Journal of Chemistry 16B: 35-37 (1978); Gupta et al., Curr Sci 46:337 (1977) (substituents at the 3- and 4- positions); DiChanna et al., J Med Chem 44: 2486-2489 (2001) (monoarylamino derivatives).

More preferably, analogs and derivatives contemplated by the present application are intended to encompass compounds having the general formula V and VI: 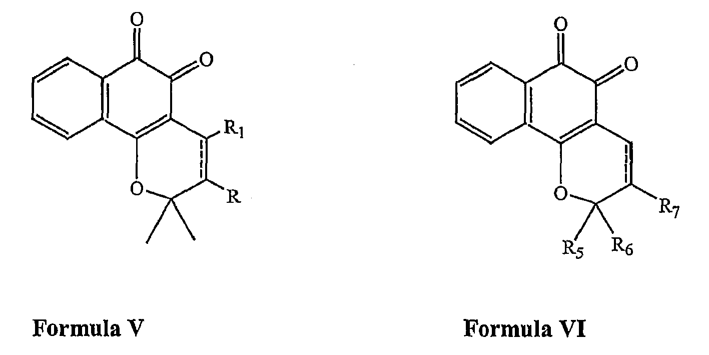 where the dotted double bond between the ring carbons represents an optional ring double bond and where R and R₁ are each independently selected from hydrogen, hydroxy, sulfhydryl, halogen, substituted alkyl, unsubstituted alkyl, substituted alkenyl, unsubstituted alkenyl, substituted aryl, unsubstituted aryl, substituted alkoxy, unsubstituted alkoxy, and salts thereof. The alkyl groups preferably have from 1 to about 15 carbon atoms, more preferably from 1 to about 10 carbon atoms, still more preferably from 1 to about 6 carbon atoms. The term alkyl refers to both cyclic and noncyclic groups. Straight or branched chain noncyclic alkyl groups are generally more preferred than cyclic groups. Straight chain alkyl groups are generally more preferred than branched. The alkenyl groups preferably have from 2 to about 15 carbon atoms, more preferably from 2 to about 10 carbon atoms, still more preferably from 2 to 6 carbon atoms. Especially preferred alkenyl groups have 3 carbon atoms (i.e., 1-propenyl or 2-propenyl), with the allyl moiety being particularly preferred. Phenyl and napthyl are generally preferred aryl groups. Alkoxy groups include those alkoxy groups having one or more oxygen linkage and preferably have from 1 to 15 carbon atoms, more preferably from 1 to about 6 carbon atoms. The substituted R and R₁ groups may be substituted at one or more available positions by one or more suitable groups such as, for example, alkyl groups having from 1 to 10 carbon atoms or from 1 to 6 carbon atoms, alkenyl groups having from 2 to 10 carbon atoms or 2 to 6 carbon atoms, aryl groups having from six to ten carbon atoms, halogen such as fluoro, chloro and bromo, and N, O and S, including heteroalkyl, e.g., heteroalkyl having one or more hetero atom linkages (and thus including alkoxy, aminoalkyl and thioalkyl) and from 1 to 10 carbon atoms or from 1 to 6 carbon atoms; and where R₅ and R₆ may be independently selected from hydroxy, C₁-C₆ alkyl, C₁-C₆ alkenyl, C₁-C₆ alkoxy, C₁-C₆ alkoxycarbonyl, --(CH₂)ₙ-aryl,-(CH₂)ₙ-heteroaryl, --(CH₂)ₙ-heterocycle, or --(CH₂)ₙ-phenyl; and R₇ is hydrogen, hydroxyl, C₁-C₆ alkyl, C₁-C₆ alkenyl, C₁-C₆ alkoxy, C₁-C₆ alkoxycarbonyl, --(CH₂)ₙ-amino, --(CH₂)ₙ-aryl,-(CH₂)ₙ-heteroaryl, --(CH₂)ₙ-heterocycle, or --(CH₂)ₙ-phenyl, wherein n is an integer from 0 to 10.

Preferred analogs and derivatives also contemplated by the invention include compounds of the following general formula VII: 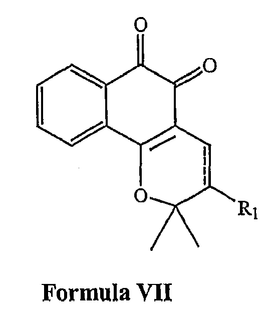 where R₁ is (CH₂)ₙ-R₂, where n is an integer from 0-10 and R₂ is hydrogen, an alkyl, an aryl, a heteroaromatic, a heterocyclic, an aliphatic, an alkoxy, an allyloxy, a hydroxyl, an amine, a thiol, an amide, or a halogen.

Analogs and derivatives also contemplated by the invention include 4-acetoxy-β-lapachone, 4-acetoxy-3-bromo-β-lapachone, 4-keto-β-lapachone, 7-hydroxy-β-lapachone, 7-methoxy-β-lapachone, 8-hydroxy-β-lapachone, 8-methoxy-β-lapachone, 8-chloro-β-lapachone, 9-chloro-β-lapachone, 8-methyl-β-lapachone and 8,9-dimethoxy-β-lapachone.

Preferred analogs and derivatives also contemplated by the invention include compounds of the following general formula VIII: 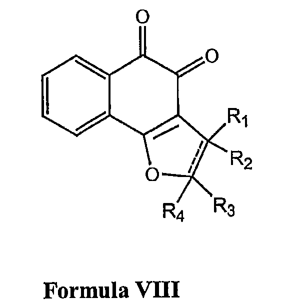 where R₁-R₄ are each, independently, selected from the group consisting of H, C₁-C₆ alkyl, C₁-C₆ alkenyl, C₁-C₆ alkoxy, C₁-C₆ alkoxycarbonyl, --(CH₂)ₙ-aryl, --(CH₂)ₙ-heteroaryl, --(CH₂)ₙ- heterocycle, or --(CH₂)ₙ-phenyl; or R₁ and R₂ combined are a single substituent selected from the above group, and R₃ and R₄ combined are a single substituent selected from the above groups, in which case ---- is a double bond.

Preferred analogs and derivatives also contemplated by this invention include dunnione and 2-ethyl-6-hydroxynaphtho[2,3-b]-furan-4,5-dione.

Preferred analogs and derivatives also contemplated by the invention include compounds of the following general formula IX: 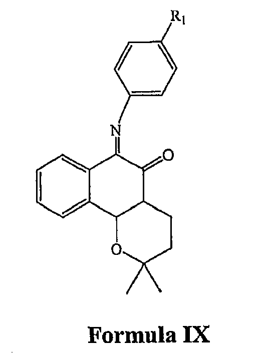 where R₁ is selected from H, CH₃, OCH₃ and NO₂.

Additional preferred β-lapachone analogs useful in the methods and kits of the invention are represented by Formula X (see also the co-owned PCT patent application entitled "NOVEL LAPACHONE COMPOUNDS AND METHODS OF USE THEREOF", PCT/US2003/037219, filed November 18, 2003, and claiming priority to U.S. provisional application no. 60/427,283, filed November 18, 2002): 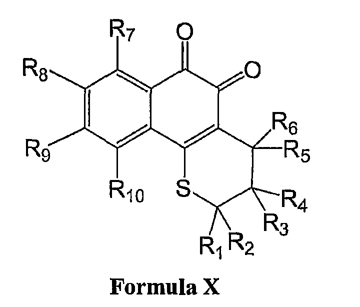 or pharmaceutically acceptable salts thereof, or a regioisomeric mixture thereof, wherein R1-R6 are each, independently, selected from the group consisting of H, OH, substituted and unsubstituted C₁-C₆ alkyl, substituted and unsubstituted C₁-C₆ alkenyl, substituted and unsubstituted C₁-C₆ alkoxy, substituted and unsubstituted C₁-C₆ alkoxycarbonyl, substituted and unsubstituted C₁-C₆ acyl, -(CH₂)ₙ-amino, -(CH₂)ₙ-aryl, -(CH₂)ₙ-heterocycle, and -(CH₂)ₙ-phenyl; or one of R1 or R2 and one of R3 or R4; or one of R3 or R4 and one of R5 or R6 form a fused ring, wherein the ring has 4-8 ring members; R7-R10 are each, independently, hydrogen, hydroxyl, halogen, substituted or unsubstituted alkyl, substituted or unsubstituted alkoxy, nitro, cyano or amide; and n is an integer from 0 to 10.

In a preferred embodiment, R1 and R2 are alkyl, R3-R6 are, independently, H, OH, halogen, alkyl, alkoxy, substituted or unsubstituted acyl, substituted alkenyl or substituted alkyl carbonyl, and R7-R10 are hydrogen. In another preferred embodiment, R1 and R2 are each methyl and R3-R10 are each hydrogen. In another preferred embodiment, R1-R4 are each hydrogen, R5 and R6 are each methyl and R7-R10 are each hydrogen.

Additional preferred β-lapachone analogs useful in the methods and kits of the invention are represented by Formula XI (see also the co-owned PCT patent application entitled "NOVEL LAPACHONE COMPOUNDS AND METHODS OF USE THEREOF", PCT/US2003/037219, filed November 18, 2003): 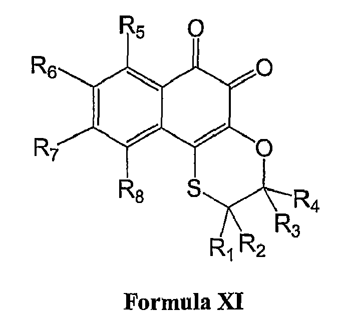 or pharmaceutically acceptable salts thereof, or a regioisomeric mixture thereof, wherein R1-R4 are each, independently, selected from the group consisting of H, OH, substituted and unsubstituted C₁-C₆ alkyl, substituted and unsubstituted C₁-C₆ alkenyl, substituted and unsubstituted C₁-C₆ alkoxy, substituted and unsubstituted C₁-C₆ alkoxycarbonyl, substituted and unsubstituted C₁-C₆ acyl, -(CH₂)ₙ-amino, -(CH₂)ₙ-aryl, -(CH₂)ₙ-heterocycle, and -(CH₂)ₙ-phenyl; or one of R1 or R2 and one of R3 or R4 form a fused ring, wherein the ring has 4-8 ring members; R5-R8 are each, independently, hydrogen, hydroxyl, halogen, substituted or unsubstituted alkyl, substituted or unsubstituted alkoxy, nitro, cyano or amide; and n is an integer from 0 to 10. In certain embodiments of Formula XI, R1, R2, R3, R4, R5, R6, R7 and R8 are not each simultaneously H.

All stereoisomers of the compounds of the instant invention are contemplated, either in admixture or in pure or substantially pure form. The definition of the compounds according to the invention embraces all possible stereoisomers (e.g., the R and S configurations for each asymmetric center) and their mixtures. It very particularly embraces the racemic forms and the isolated optical isomers having a specified activity. The racemic forms can be resolved by physical methods, such as, for example, fractional crystallization, separation or crystallization of diastereomeric derivatives or separation by chiral column chromatography. The individual optical isomers can be obtained from the racemates by conventional methods, such as, for example, salt formation with an optically active acid followed by crystallization. Furthermore, all geometric isomers, such as E- and Z-configurations at a double bond, are within the scope of the invention unless otherwise stated. Certain compounds of this invention may exist in tautomeric forms. All such tautomeric forms of the compounds are considered to be within the scope of this invention unless otherwise stated. The present invention also includes one or more regioisomeric mixtures of an analog or derivative of β-Lapachone.

As used herein, the term "salt" is a pharmaceutically acceptable salt and can include acid addition salts including hydrochlorides, hydrobromides, phosphates, sulphates, hydrogen sulphates, alkylsulphonates, arylsulphonates, acetates, benzoates, citrates, maleates, fumarates, succinates, lactates, and tartrates; alkali metal cations such as Na, K, Li, alkali earth metal salts such as Mg or Ca, or organic amine salts.

As used herein, the term "metabolite" means a product of metabolism of β-lapachone, or a pharmaceutically acceptable salt, analog or derivative thereof, that exhibits a similar activity in vivo to β-lapachone.

As used herein, the term "prodrug" means a compound of the present invention covalently linked to one or more pro-moieties, such as an amino acid moiety or other water solubilizing moiety. A compound of the present invention may be released from the pro-moiety via hydrolytic, oxidative, and/or enzymatic release mechanisms. In an embodiment, a prodrug composition of the present invention exhibits the added benefit of increased aqueous solubility, improved stability, and improved pharmacokinetic profiles. The pro-moiety may be selected to obtain desired prodrug characteristics. For example, the pro-moiety, e.g., an amino acid moiety or other water solubilizing moiety may be selected based on solubility, stability, bioavailability, and/or in vivo delivery or uptake.

### II. Methods of Treatment

As used herein, a "subject" can be any mammal, e.g., a human, a primate, mouse, rat, dog, cat, cow, horse, pig, sheep, goat, camel. In a preferred aspect, the subject is a human.

As used herein, a "subject in need thereof" is a subject having a cell proliferative disorder of the lung, or a subject having an increased risk of developing a cell proliferative disorder of the lung relative to the population at large. In one aspect, a subject in need thereof has a precancerous condition of the lung. In a preferred aspect, a subject in need thereof has lung cancer. In an aspect, the subject may be suffering from a known (i.e., diagnosed) condition characterized by cell hyperproliferation (*e.g.,* cancer) of the lung.

As used herein, the term "cell proliferative disorder" refers to conditions in which unregulated or abnormal growth, or both, of cells can lead to the development of an unwanted condition or disease, which may or may not be cancerous. In one aspect, a cell proliferative disorder includes, for example, lung cancer and precancerous conditions of the lung. A "cell proliferative disorder of the lung" is a cell proliferative disorder involving cells of the lung. In one aspect, a cell proliferative disorder includes a pre-cancer or precancerous conditionof the lung. In one aspect, a cell proliferative disorder of the lung includes a non-cancerous cell proliferative disorder of the lung. In another aspect, a cell proliferative disorder includes lung cancer, including metastatic lesions in other tissues or organs distant from the primary tumor site. In one aspect, a "precancer cell" or "precancerous cell" is a cell manifesting a cell proliferative disorder that is a precancer or a precancerous condition. In another aspect, a "cancer cell" or "cancerous cell" is a cell manifesting a cell proliferative disorder that is a cancer. Any reproducible means of measurement may be used to identify cancer cells or precancerous cells. In a preferred aspect, cancer cells or precancerous cells are identified by histological typing or grading of a tissue sample (e.g., a biopsy sample). In another aspect, cancer cells or precancerous cells are identified through the use of appropriate molecular markers.

In a preferred aspect, the cell proliferative disorder of the lung is lung cancer. In a preferred aspect, compositions of the present invention may be used to treat lung cancer or cell proliferative disorders of the lung. In one aspect, lung cancer includes all forms of cancer of the lung. Cancers to be treated include but are not limited to sarcoma, carcinoma, and adenocarcinoma. In another aspect, lung cancer includes malignant lung neoplasms, carcinoma *in situ,* typical carcinoid tumors, and atypical carcinoid tumors. In another aspect, lung cancer includes small cell lung cancer ("SCLC"), non-small cell lung cancer ("NSCLC"), squamous cell carcinoma, adenocarcinoma, small cell carcinoma, large cell carcinoma, adenosquamous cell carcinoma, and mesothelioma. In another aspect, lung cancer includes "scar carcinoma," bronchioalveolar carcinoma, giant cell carcinoma, spindle cell carcinoma, and large cell neuroendocrine carcinoma. In another aspect, lung cancer includes lung neoplasms having histologic and ultrastructual heterogeneity (e.g., mixed cell types). In one aspect, lung cancer includes mixed small cell/large cell carcinoma.

In one aspect, cell proliferative disorders of the lung include all forms of cell proliferative disorders affecting lung cells. In one aspect, cell proliferative disorders of the lung include lung cancer and precancerous conditions of the lung. In one aspect, cell proliferative disorders of the lung include hyperplasia, metaplasia, and dysplasia of the lung. In one aspect, cell proliferative disorders to be treated include sporadic and hereditary cell proliferative disorders of the lung. In one aspect, cell proliferative disorders of the lung include benign tumors of the lung. In another aspect, cell proliferative disorders of the lung include asbestos-induced hyperplasia, squamous metaplasia, and benign reactive mesothelial metaplasia. In another aspect, cell proliferative disorders of the lung include replacement of columnar epithelium with stratified squamous epithelium, and mucosal dysplasia. In another aspect, individuals exposed to inhaled injurious environmental agents such as cigarette smoke and asbestos may be at increased risk for developing cell proliferative disorders of the lung. In another aspect, prior lung diseases that may predispose individuals to development of cell proliferative disorders of the lung include chronic interstitial lung disease, necrotizing pulmonary disease, scleroderma, rheumatoid disease, sarcoidosis, interstitial pneumonitis, tuberculosis, repeated pneumonias, idiopathic pulmonary fibrosis, granulomata, asbestosis, fibrosing alveolitis, and Hodgkin's disease.

In one aspect, a lung cancer that is to be treated has arisen in a subject equal to or older than 30 years old, or a subject younger than 30 years old. In one aspect, a lung cancer that is to be treated has arisen in a subject equal to or older than 50 years old, or a subject younger than 50 years old. In one aspect, a lung cancer that is to be treated has arisen in a subject equal to or older than 70 years old, or a subject younger than 70 years old. In one aspect, a lung cancer that is to be treated has been typed to identify a familial or spontaneous mutation in p53, Rb, CDKN2A (P16INK4A), FHIT, myc, ras, TP73, MADH2, MADH4, PPP2R1b, or PTEN. In another aspect, a lung cancer that is to be treated is associated with a GSTM1 null allele. In another aspect, a lung cancer that is to be treated is associated with a mutation selected from the group consisting of del(3p), del(9p) and del(1p36). In one aspect, a lung cancer that is to be treated is associated with elevated levels of CEA (carcinoembryonic antigen) or NSE (neuron-specific enolase), or an upregulation of one or more components of telomerase. In another aspect, a lung cancer that is to be treated is associated with an increased level of a marker selected from the group consisting of MOC-1, MOC-21, MOC-31, MOC-32, and MOC-52. In one aspect, a lung cancer that is to be treated includes a localized tumor of the lung. In one aspect, a lung cancer that is to be treated includes a tumor of the lung that is associated with a negative regional lymph node biopsy. In one aspect, a lung cancer that is to be treated includes a tumor of the lung that is associated with a positive regional lymph node biopsy. In another aspect, a lung cancer that is to be treated includes a tumor of the lung that has been typed as having nodal negative status (e.g., node-negative) or nodal positive status (e.g., node-positive). In another aspect, a lung cancer that is to be treated includes a tumor of the lung that has metastasized to other locations in the body. In one aspect, a lung cancer that is to be treated is classified as having metastasized to a location selected from the group consisting of lymph node, stomach, bile duct, lung, liver, bone, and brain. In another aspect a lung cancer that is to be treated is classified according to a characteristic selected from the group consisting of metastatic, limited stage, extensive stage, unresectable, resectable, localized, regional, local-regional, locally advanced, distant, multicentric, bilateral, ipsilateral, contralateral, newly diagnosed, recurrent, and inoperable.

In one aspect, a lung cancer that is to be treated has been staged according to the American Joint Committee on Cancer (AJCC) TNM classification system, where the tumor (T) has been assigned a stage of Tis, T1, T2, T3,T4; and where the regional lymph nodes (N) have been assigned a stage of NX, N0, N1, N2, N2a, N2b, N3, N3a, N3b, or N3c; and where distant metastasis (M) has been assigned a stage of MX, M0, or M1. In another aspect, a lung cancer that is to be treated has been staged according to an American Joint Committee on Cancer (AJCC) classification as Stage 0,I, IA, IB, II, IIA, IIB, III, IIIA, IIIB, IIIC and IV lung cancer. In another aspect, a lung cancer that is to be treated has been assigned a grade according to an AJCC classification as Grade GX (e.g., grade cannot be assessed), Grade 1, Grade 2, Grade 3 or Grade 4.

In one aspect, a lung cancer that is to be treated includes a tumor that has been determined to be less than or equal to about 3 centimeters in diameter. In another aspect, a lung cancer that is to be treated includes a tumor that has been determined to be from about 3 to about 5 centimeters in diameter. In another aspect, a lung cancer that is to be treated includes a tumor that has been determined to be greater than or equal to about 3 centimeters in diameter. In another aspect, a lung cancer that is to be treated includes a tumor that has been determined to be greater than 5 centimeters in diameter. In another aspect, a lung cancer that is to be treated is classified by microscopic appearance as well differentiated, moderately differentiated, poorly differentiated, or undifferentiated. In another aspect, a lung cancer that is to be treated is classified by microscopic appearance with respect to mitosis count (e.g., amount of cell division) or nuclear pleiomorphism (e.g., change in cells). In another aspect, a lung cancer that is to be treated is classified by microscopic appearance as being associated with areas of necrosis (e.g., areas of dying or degenerating cells). In one aspect, a lung cancer that is to be treated is classified as having an abnormal karyotype, having an abnormal number of chromosomes, or having one or more chromosomes that are abnormal in appearance. In one aspect, a lung cancer that is to be treated is classified as being aneuploid, triploid, tetraploid, or as having an altered ploidy. In one aspect, a lung cancer that is to be treated is classified as having a chromosomal translocation, or a deletion or duplication of an entire chromosome, or a region of deletion, duplication or amplification of a portion of a chromosome.

In one aspect, a lung cancer that is to be treated is evaluated by DNA cytometry, flow cytometry, or image cytometry. In one aspect, a lung cancer that is to be treated has been typed as having 10%, 20%, 30%, 40%, 50%, 60%, 70%, 80%, or 90% of cells in the synthesis stage of cell division (e.g., in S phase of cell division). In one aspect, a lung cancer that is to be treated has been typed as having a low S-phase fraction or a high S-phase fraction.

As used herein, a "normal cell" is a cell that cannot be classified as part of a "cell proliferative disorder." In one aspect, a normal cell lacks unregulated or abnormal growth, or both, that can lead to the development of an unwanted condition or disease. Preferably, a normal cell possesses normally functioning cell cycle checkpoint control mechanisms.

As used herein, "contacting a cell" refers to a condition in which a compound or other composition of matter is in direct contact with a cell, or is close enough to induce a desired biological effect in a cell.

As used herein, "monotherapy" refers to administration of a single active or therapeutic compound to a subject in need thereof. Preferably, monotherapy will involve administration of a therapeutically effective amount of an active compound. For example, β-lapachone monotherapy for cancer comprises administration of a therapeutically effective amount of β-lapachone, or a pharmaceutically acceptable salt, prodrug, metabolite, analog or derivative thereof, to a subject in need of treatment of cancer. Monotherapy may be contrasted with combination therapy, in which a combination of multiple active compounds is administered, preferably with each component of the combination present in a therapeutically effective amount. In one aspect, β-lapachone montherapy is more effective than combination therapy in inducing a desired biological effect.

In one aspect, combination therapy includes β-lapachone with taxol; β-lapachone with docetaxel; β-lapachone with vincristin; β-lapachone with vinblastin; β-lapachone with nocodazole; β-lapachone with teniposide; β-lapachone with etoposide; β-lapachone with adriamycin; β-lapachone with epothilone; β-lapachone with navelbine; β-lapachone with camptothecin; β-lapachone with daunorubicin; β-lapachone with dactinomycin; β-lapachone, with mitoxantrone; β-lapachone with amsacrine; β-lapachone with epirubicin; or β-lapachone, with idarubicin. In a preferred aspect, combination therapy includes β-lapachone with gemcitabine. In another aspect, combination therapy includes reduced β-lapachone with taxol; reduced β-lapachone with docetaxel; reduced β-lapachone with vincristin; reduced β-lapachone, with vinblastin; reduced β-lapachone with nocodazole; reduced β-lapachone with teniposide; reduced β-lapachone with etoposide; reduced β-lapachone with adriamycin; reduced β-lapachone with epothilone; reduced β-lapachone with navelbine; reduced β-lapachone with camptothecin; reduced β-lapachone with daunorubicin; reduced β-lapachone with dactinomycin; reduced β-lapachone with mitoxantrone; reduced β-lapachone with amsacrine; reduced β-lapachone with epirubicin; or reduced β-lapachone with idarubicin. In a preferred aspect, combination therapy includes reduced β-lapachone with gemcitabine.

As used herein, "treating" describes the management and care of a patient for the purpose of combating a disease, condition, or disorder and includes the administration of a compound of the present invention to prevent the onset of the symptoms or complications, alleviating the symptoms or complications, or eliminating the disease, condition or disorder.

In one aspect, treating a lung cancer of the present invention results in a reduction in size of a tumor. A reduction in size of a tumor may also be referred to as "tumor regression." Preferably, after treatment, tumor size is reduced by 5% or greater relative to its size prior to treatment; more preferably, tumor size is reduced by 10% or greater; more preferably, reduced by 20% or greater; more preferably, reduced by 30% or greater; more preferably, reduced by 40% or greater; even more preferably, reduced by 50% or greater; and most preferably, reduced by greater than 75% or greater. Size of a tumor may be measured by any reproducible means of measurement. In a preferred aspect, size of a tumor may be measured as a diameter of the tumor.

In another aspect, treating a lung cancer of the present invention results in a reduction in tumor volume. Preferably, after treatment, tumor volume is reduced by 5% or greater relative to its size prior to treatment; more preferably, tumor volume is reduced by 10% or greater; more preferably, reduced by 20% or greater; more preferably, reduced by 30% or greater; more preferably, reduced by 40% or greater; even more preferably, reduced by 50% or greater; and most preferably, reduced by greater than 75% or greater. Tumor volume may be measured by any reproducible means of measurement.

In another aspect, treating a lung cancer of the present invention results in a decrease in number of tumors. Preferably, after treatment, tumor number is reduced by 5% or greater relative to number prior to treatment; more preferably, tumor number is reduced by 10% or greater; more preferably, reduced by 20% or greater; more preferably, reduced by 30% or greater; more preferably, reduced by 40% or greater; even more preferably, reduced by 50% or greater; and most preferably, reduced by greater than 75%. Number of tumors may be measured by any reproducible means of measurement. In a preferred aspect, number of tumors may be measured by counting tumors visible to the naked eye or at a specified magnification. In a preferred aspect, the specified magnification is 2x, 3x, 4x, 5x, 10x, or 50x.

In another aspect, treating a lung cancer of the present invention results in a decrease in number of metastatic lesions in other tissues or organs distant from the primary tumor site. Preferably, after treatment, the number of metastatic lesions is reduced by 5% or greater relative to number prior to treatment; more preferably, the number of metastatic lesions is reduced by 10% or greater; more preferably, reduced by 20% or greater; more preferably, reduced by 30% or greater; more preferably, reduced by 40% or greater; even more preferably, reduced by 50% or greater; and most preferably, reduced by greater than 75%. The number of metastatic lesions may be measured by any reproducible means of measurement. In a preferred aspect, the number of metastatic lesions may be measured by counting metastatic lesions visible to the naked eye or at a specified magnification. In a preferred aspect, the specified magnification is 2x, 3x, 4x, 5x, 10x, or 50x.

In another aspect, treating a lung cancer of the present invention results in an increase in average survival time of a population of treated subjects in comparison to a population receiving carrier alone. Preferably, the average survival time is increased by more than 30 days; more preferably, by more than 60 days; more preferably, by more than 90 days; and most preferably, by more than 120 days. An increase in average survival time of a population may be measured by any reproducible means. In a preferred aspect, an increase in average survival time of a population may be measured, for example, by calculating for a population the average length of survival following initiation of treatment with an active compound. In an another preferred aspect, an increase in average survival time of a population may also be measured, for example, by calculating for a population the average length of survival following completion of a first round of treatment with an active compound.

In another aspect, treating a lung cancer of the present invention results in an increase in average survival time of a population of treated subjects in comparison to a population of untreated subjects. Preferably, the average survival time is increased by more than 30 days; more preferably, by more than 60 days; more preferably, by more than 90 days; and most preferably, by more than 120 days. An increase in average survival time of a population may be measured by any reproducible means. In a preferred aspect, an increase in average survival time of a population may be measured, for example, by calculating for a population the average length of survival following initiation of treatment with an active compound. In an another preferred aspect, an increase in average survival time of a population may also be measured, for example, by calculating for a population the average length of survival following completion of a first round of treatment with an active compound.

In another aspect, treating a lung cancer of the present invention results in increase in average survival time of a population of treated subjects in comparison to a population receiving monotherapy with a drug that is not β-lapachone, or a pharmaceutically acceptable salt, metabolite, analog or derivative thereof. Preferably, the average survival time is increased by more than 30 days; more preferably, by more than 60 days; more preferably, by more than 90 days; and most preferably, by more than 120 days. An increase in average survival time of a population may be measured by any reproducible means. In a preferred aspect, an increase in average survival time of a population may be measured, for example, by calculating for a population the average length of survival following initiation of treatment with an active compound. In an another preferred aspect, an increase in average survival time of a population may also be measured, for example, by calculating for a population the average length of survival following completion of a first round of treatment with an active compound.

In another aspect, treating a lung cancer of the present invention results in a decrease in the mortality rate of a population of treated subjects in comparison to a population receiving carrier alone. In another aspect, treating lung cancer results in a decrease in the mortality rate of a population of treated subjects in comparison to an untreated population. In a further aspect, treating lung cancer results a decrease in the mortality rate of a population of treated subjects in comparison to a population receiving monotherapy with a drug that is not β-lapachone, or a pharmaceutically acceptable salt, metabolite, analog or derivative thereof. Preferably, the mortality rate is decreased by more than 2%; more preferably, by more than 5%; more preferably, by more than 10%; and most preferably, by more than 25%. In a preferred aspect, a decrease in the mortality rate of a population of treated subjects may be measured by any reproducible means. In another preferred aspect, a decrease in the mortality rate of a population may be measured, for example, by calculating for a population the average number of disease-related deaths per unit time following initiation of treatment with an active compound. In another preferred aspect, a decrease in the mortality rate of a population may also be measured, for example, by calculating for a population the average number of disease-related deaths per unit time following completion of a first round of treatment with an active compound.

In another aspect, treating a lung cancer of the present invention results in a decrease in tumor growth rate. Preferably, after treatment, tumor growth rate is reduced by at least 5% relative to number prior to treatment; more preferably, tumor growth rate is reduced by at least 10%; more preferably, reduced by at least 20%; more preferably, reduced by at least 30%; more preferably, reduced by at least 40%; more preferably, reduced by at least 50%; even more preferably, reduced by at least 50%; and most preferably, reduced by at least 75%. Tumor growth rate may be measured by any reproducible means of measurement. In a preferred aspect, tumor growth rate is measured according to a change in tumor diameter per unit time.

In another aspect, treating a lung cancer of the present invention results in a decrease in tumor regrowth. Preferably, after treatment, tumor regrowth is less than 5%; more preferably, tumor regrowth is less than 10%; more preferably, less than 20%; more preferably, less than 30%; more preferably, less than 40%; more preferably, less than 50%; even more preferably, less than 50%; and most preferably, less than 75%. Tumor regrowth may be measured by any reproducible means of measurement. In a preferred aspect, tumor regrowth is measured, for example, by measuring an increase in the diameter of a tumor after a prior tumor shrinkage that followed treatment. In another preferred aspect, a decrease in tumor regrowth is indicated by failure of tumors to reoccur after treatment has stopped.

In another aspect, treating or preventing a cell proliferative disorder of the present invention results in a reduction in the rate of cellular proliferation. Preferably, after treatment, the rate of cellular proliferation is reduced by at least 5%; more preferably, by at least 10%; more preferably, by at least 20%; more preferably, by at least 30%; more preferably, by at least 40%; more preferably, by at least 50%; even more preferably, by at least 50%; and most preferably, by at least 75%. The rate of cellular proliferation may be measured by any reproducible means of measurement. In a preferred aspect, the rate of cellular proliferation is measured, for example, by measuring the number of dividing cells in a tissue sample per unit time.

In another aspect, treating or preventing a cell proliferative disorder of the present invention results in a reduction in the proportion of proliferating cells. Preferably, after treatment, the proportion of proliferating cells is reduced by at least 5%; more preferably, by at least 10%; more preferably, by at least 20%; more preferably, by at least 30%; more preferably, by at least 40%; more preferably, by at least 50%; even more preferably, by at least 50%; and most preferably, by at least 75%. The proportion of proliferating cells may be measured by any reproducible means of measurement. In a preferred aspect, the proportion of proliferating cells is measured, for example, by quantifying the number of dividing cells relative to the number of nondividing cells in a tissue sample. In another preferred aspect, the proportion of proliferating cells is equivalent to the mitotic index.

In another aspect, treating or preventing a cell proliferative disorder of the present invention results in a decrease in size of an area or zone of cellular proliferation. Preferably, after treatment, size of an area or zone of cellular proliferation is reduced by at least 5% relative to its size prior to treatment; more preferably, reduced by at least 10%; more preferably, reduced by at least 20%; more preferably, reduced by at least 30%; more preferably, reduced by at least 40%; more preferably, reduced by at least 50%; even more preferably, reduced by at least 50%; and most preferably, reduced by at least 75%. Size of an area or zone of cellular proliferation may be measured by any reproducible means of measurement. In a preferred aspect, size of an area or zone of cellular proliferation may be measured as a diameter or width of an area or zone of cellular proliferation.

In another aspect, treating or preventing a cell proliferative disorder of the present invention results in a decrease in the number or proportion of cells having an abnormal appearance or morphology. Preferably, after treatment, the number of cells having an abnormal morphology is reduced by at least 5% relative to its size prior to treatment; more preferably, reduced by at least 10%; more preferably, reduced by at least 20%; more preferably, reduced by at least 30%; more preferably, reduced by at least 40%; more preferably, reduced by at least 50%; even more preferably, reduced by at least 50%; and most preferably, reduced by at least 75%. An abnormal cellular appearance or morphology may be measured by any reproducible means of measurement. In one aspect, an abnormal cellular morphology is measured by microscopy, e.g., using an inverted tissue culture microscope. In one aspect, an abnormal cellular morphology takes the form of nuclear pleiomorphism.

As used herein, the term "selectively" means tending to occur at a higher frequency in one population than in another population. In one aspect, the compared populations are cell populations. In a preferred aspect, β-lapachone, or a pharmaceutically acceptable salt, metabolite, analog or derivative thereof, acts selectively on a cancer or precancer cell but not on a normal cell. In another preferred aspect, a compound of the present invention, or a pharmaceutically acceptable salt, prodrug, metabolite, analog or derivative thereof, acts selectively to modulate one molecular target (e.g., E2F-1) but does not significantly modulate another molecular target (e.g., Protein Kinase C). In another preferred aspect, the invention provides a method for selectively inhibiting the activity of an enzyme, such as a kinase. Preferably, an event occurs selectively in population A relative to population B if it occurs greater than two times more frequently in population A as compared to population B. More preferably, an event occurs selectively if it occurs greater than five times more frequently in population A. More preferably, an event occurs selectively if it occurs greater than ten times more frequently in population A; more preferably, greater than fifty times; even more preferably, greater than 100 times; and most preferably, greater than 1000 times more frequently in population A as compared to population B. For example, cell death would be said to occur selectively in cancer cells if it occurred greater than twice as frequently in cancer cells as compared to normal cells.

In a preferred aspect, a compound of the present invention or a pharmaceutically acceptable salt, prodrug, metabolite, analog or derivative thereof, modulates the activity of a molecular target (e.g., E2F-1). In one aspect, modulating refers to stimulating or inhibiting an activity of a molecular target. Preferably, a compound of the present invention modulates the activity of a molecular target if it stimulates or inhibits the activity of the molecular target by at least 10% relative to the activity of the molecular target under the same conditions but lacking only the presence of said compound. More preferably, a compound of the present invention modulates the activity of a molecular target if it stimulates or inhibits the activity of the molecular target by at least 25%, at least 50%, at least 2-fold, at least 5-fold, at least 10-fold, at least 20-fold, at least 50-fold, at least 100-fold relative to the activity of the molecular target under the same conditions but lacking only the presence of said compound. The activity of a molecular target may be measured by any reproducible means. The activity of a molecular target may be measured in vitro or in vivo. For example, the activity of a molecular target may be measured in vitro by an enzymatic activity assay or a DNA binding assay, or the activity of a molecular target may be measured in vivo by assaying for expression of a reporter gene.

In one aspect, a compound of the present invention, or a pharmaceutically acceptable salt, prodrug, metabolite, analog or derivative thereof, does not significantly modulate the activity of a molecular target if the addition of the compound stimulates or inhibits the activity of the molecular target by less than 10% relative to the activity of the molecular target under the same conditions but lacking only the presence of said compound.

As used herein, the term "isozyme selective" means preferential inhibition or stimulation of a first isoform of an enzyme in comparison to a second isoform of an enzyme (*e.g.,* preferential inhibition or stimulation of a kinase isozyme alpha in comparison to a kinase isozyme beta). Preferably, a compound of the present invention demonstrates a minimum of a four fold differential, preferably a ten fold differential, more preferably a fifty fold differential, in the dosage required to achieve a biological effect. Preferably, a compound of the present invention demonstrates this differential across the range of inhibition, and the differential is exemplified at the IC₅₀, *i.e.,* a 50% inhibition, for a molecular target of interest.

In a preferred embodiment, administering β-lapachone, or a pharmaceutically acceptable salt, prodrug, metabolite, analog or derivative thereof, to a cell or a subject in need thereof results in modulation (i.e., stimulation or inhibition) of an activity of a member of the E2F family of transcription factors (e.g., E2F-1, E2F-2, or E2F-3). As used herein, an activity of a member of the E2F family of transcription factors refers to any biological function or activity that is carried out by an E2F family member. For example, a function of E2F-1 includes binding of E2F-1 to its cognate DNA sequences. Other functions of E2F-1 include migrating to the cell nucleus and activating transcription.

In one aspect, treating lung cancer or a cell proliferative disorder results in cell death, and preferably, cell death results in a decrease of at least 10% in number of cells in a population. More preferably, cell death means a decrease of at least 20%; more preferably, a decrease of at least 30%; more preferably, a decrease of at least 40%; more preferably, a decrease of at least 50%; most preferably, a decrease of at least 75%. Number of cells in a population may be measured by any reproducible means. In one aspect, number of cells in a population is measured by fluorescence activated cell sorting (FACS). In another aspect, number of cells in a population is measured by immunofluorescence microscopy. In another aspect, number of cells in a population is measured by light microscopy. In another aspect, methods of measuring cell death are as shown in Li et al., (2003) Proc Natl Acad Sci U S A. 100(5): 2674-8. In a preferred aspect, cell death results from apoptosis.

In a preferred aspect, an effective amount of β-lapachone, or a pharmaceutically acceptable salt, metabolite, analog or derivative thereof is not significantly cytotoxic to normal cells. A therapeutically effective amount of a compound is not significantly cytotoxic to normal cells if administration of the compound at a therapeutically effective amount does not induce apoptosis in greater than 10% of normal cells. A therapeutically effective amount of a compound does not significantly affect the viability of normal cells if administration of the compound at a therapeutically effective amount does not induce cell death in greater than 10% of normal cells.

In one aspect, activating refers to placing one or more compositions of matter (e.g., protein or nucleic acid) in a state suitable for carrying out a desired biological function. In one aspect, a composition of matter capable of being activated also has an unactivated state. In one aspect, an activated composition of matter may have an inhibitory or stimulatory biological function, or both.

In one aspect, elevation refers to an increase in a desired biological activity of a composition of matter (*e.g.,* a protein or a nucleic acid). In one aspect, elevation may occur through an increase in concentration of a composition of matter.

In one aspect, stimulation of unscheduled expression of a checkpoint molecule by β-lapachone, or a pharmaceutically acceptable salt, metabolite, analog or derivative thereof, triggers cell death in cells with defective checkpoints, a hallmark of cancer and pre-cancer cells. In one aspect, contacting a cell with β-lapachone, or a pharmaceutically acceptable salt, metabolite, analog or derivative thereof, stimulates unscheduled expression of the checkpoint molecule E2F.

In one aspect, contacting a cell with β-lapachone, or a pharmaceutically acceptable salt, metabolite, analog or derivative thereof, results in activation of an E2F checkpoint pathway. Preferably, administering to a subject in need thereof β-lapachone, or a pharmaceutically acceptable salt, metabolite, analog or derivative thereof, results in activation of an E2F checkpoint pathway. In a preferred aspect, E2F pathway activity is increased by more than 10%; more than 25%; more than 50%; more than 2-fold; more than 5-fold; and most preferably, by more than 10-fold. In another preferred aspect, E2F activity is increased by more than 10%; more than 25%; more than 50%; more than 2-fold; more than 5-fold; and most preferably, by more than 10-fold. Methods of measuring induction of E2F activity and elevation of E2F levels are as shown in Li et al., (2003) Proc Natl Acad Sci USA. 100(5): 2674-8.

In one aspect, contacting a cell with β-lapachone, or a pharmaceutically acceptable salt, metabolite, analog or derivative thereof, results in elevation of an E2F transcription factor. Preferably, administering to a subject in need thereof β-lapachone, or a pharmaceutically acceptable salt, metabolite, analog or derivative thereof, results in elevation of an E2F transcription factor.

In one aspect, contacting a cell with β-lapachone, or a pharmaceutically acceptable salt, metabolite, analog or derivative thereof, results in elevation of an E2F transcription factor selectively in lung cancer cells but not in normal cells. Preferably, administering to a subject in need thereof β-lapachone, or a pharmaceutically acceptable salt, metabolite, analog or derivative thereof, results in elevation of an E2F transcription factor selectively in lung cancer cells but not in normal cells.

In one aspect, contacting a cell with β-lapachone, or a pharmaceutically acceptable salt, metabolite, analog or derivative thereof, stimulates unscheduled activation of an E2F transcription factor. Preferably, administering to a subject in need thereof β-lapachone, or a pharmaceutically acceptable salt, metabolite, analog or derivative thereof, stimulates unscheduled activation of an E2F transcription factor.

In one aspect, contacting a cell with β-lapachone, or a pharmaceutically acceptable salt, metabolite, analog or derivative thereof, stimulates unscheduled activation of an E2F transcription factor selectively in lung cancer cells but not in normal cells. Preferably, administering to a subject in need thereof β-lapachone, or a pharmaceutically acceptable salt, metabolite, analog or derivative thereof, stimulates unscheduled activation of an E2F transcription factor selectively in lung cancer cells but not in normal cells.

In normal cells with their intact regulatory mechanisms, imposed expression of a checkpoint molecule (e.g., as induced by contacting a cell with β-lapachone, or a pharmaceutically acceptable salt, metabolite, analog or derivative thereof) results in an expression pattern that is not reported to be of substantial consequence. In contrast, cancer and pre-cancer cells have defective mechanisms, which result in unchecked or persistent expression, or both, of unscheduled checkpoint molecules, e.g., E2F, leading to selective cell death in cancer and pre-cancer cells. The present invention includes and provides for the unchecked or persistent expression, or both, of unscheduled checkpoint molecules by the administration of β-lapachone, or a pharmaceutically acceptable salt, metabolite, analog or derivative thereof.

In one aspect, contacting a cell with β-lapachone, or a pharmaceutically acceptable salt, metabolite, analog or derivative thereof, results in activation of one or more cell cycle checkpoints. Preferably, administering to a subject in need thereof β-lapachone, or a pharmaceutically acceptable salt, metabolite, analog or derivative thereof, results in activation of one or more cell cycle checkpoints.

In one aspect, contacting a cell with β-lapachone, or a pharmaceutically acceptable salt, metabolite, analog or derivative thereof, results in activation of one or more cell cycle checkpoint pathways. Preferably, administering to a subject in need thereof β-lapachone, or a pharmaceutically acceptable salt, metabolite, analog or derivative thereof, results in activation of one or more cell cycle checkpoint pathways.

In one aspect, contacting a cell with β-lapachone, or a pharmaceutically acceptable salt, metabolite, analog or derivative thereof, results in activation of one or more cell cycle checkpoint regulators. Preferably, administering to a subject in need thereof β-lapachone, or a pharmaceutically acceptable salt, metabolite, analog or derivative thereof, results in activation of one or more cell cycle checkpoint regulators.

In one aspect, contacting a cell with β-lapachone, or a pharmaceutically acceptable salt, metabolite, analog or derivative thereof, induces or activates cell death selectively in lung cancer cells. Preferably, administering to a subject in need thereof β-lapachone, or a pharmaceutically acceptable salt, metabolite, analog or derivative thereof, induces or activates cell death selectively in lung cancer cells. In another aspect, contacting a cell with β-lapachone, or a pharmaceutically acceptable salt, metabolite, analog or derivative thereof, induces cell death selectively in one or more cells affected by a cell proliferative disorder of the lung. Preferably, administering to a subject in need thereof β-lapachone, or a pharmaceutically acceptable salt, metabolite, analog or derivative thereof, induces cell death selectively in one or more cells affected by a cell proliferative disorder of the lung.

In a preferred aspect, the present invention relates to a method of treating or preventing cancer by administering β-lapachone, or a pharmaceutically acceptable salt, metabolite, analog or derivative thereof to a subject in need thereof, where administration of the β-lapachone, or a pharmaceutically acceptable salt, metabolite, analog or derivative thereof results in one or more of the following: accumulation of cells in G1 and/or S phase of the cell cycle, cytotoxicity via cell death in cancer cells but not in normal cells, antitumor activity in animals with a therapeutic index of at least 2, and acitvation of a cell cycle checkpoint (e.g., activation or elevation of a member of the E2F family of transcription factors). As used herein, "therapeutic index" is the maximum tolerated dose divided by the efficacious dose.

In additional aspects, β-lapachone, or a pharmaceutically acceptable salt, metabolite, analog or derivative thereof, can be administered in combination with a chemotherapeutic agent. Exemplary chemotheraputics with activity against cell proliferative disorders, such as lung cancer, are known to those of ordinary skill in the art, and may be found in reference texts such as the Physician's Desk Reference, 59th Edition, Thomson PDR (2005). For example, the chemotherapeutic agent can be a taxane, an aromatase inhibitor, an anthracycline, a microtubule targeting drug, a topoisomerase poison drug, a targeted monoclonal or polyconal antibody, an inhibitor of a molecular target or enzyme (e.g., a kinase inhibitor), or a cytidine analogue drug. In preferred aspects, the chemotherapeutic agent can be, but is not restricted to, tamoxifen, raloxifene, anastrozole, exemestane, letrozole, HERCEPTIN^{®} (trastuzumab), GLEEVEC^{®} (imatanib), TAXOL^{®} (paclitaxel), IRESSA^{®} (gefitinib), TARCEVA™ (erlotinib), cyclophosphamide, lovastatin, minosine, araC, 5-fluorouracil (5-FU), methotrexate (MTX), TAXOTERE^{®} (docetaxel), ZOLADEX^{®} (goserelin), AVASTIN™ (bevacizumab), vincristin, vinblastin, nocodazole, teniposide, etoposide, epothilone, navelbine, camptothecin, daunonibicin, dactinomycin, mitoxantrone, amsacrine, doxorubicin (adriamycin), epirubicin or idarubicin or agents listed in www.cancer.org/docroot/cdg/cdg_0.asp. In another aspect, the chemotherapeutic agent can be a cytokine such as G-CSF (granulocyte colony stimulating factor). In another aspect, β-lapachone, or a pharmaceutically acceptable salt, metabolite, analog or derivative thereof may be administered in combination with radiation therapy. In yet another aspect, β-lapachone, or a pharmaceutically acceptable salt, metabolite, analog or derivative thereof may be administered in combination with standard chemotherapy combinations such as, but not restricted to, CMF (cyclophosphamide, methotrexate and 5-fluorouracil), CAF (cyclophosphamide, adriamycin and 5-fluorouracil), AC (adriamycin and cyclophosphamide), FEC (5-fluorouracil, epirubicin, and cyclophosphamide), ACT or ATC (adriamycin, cyclophosphamide, and paclitaxel), or CMFP (cyclophosphamide, methotrexate, 5-fluorouracil and prednisone).

In a preferred aspect, a cell proliferative disorder of the lung, such as lung cancer, is treated by administering to a patient in need thereof a therapeutically effective amount of β-lapachone, or a pharmaceutically acceptable salt, metabolite, analog or derivative thereof, in combination with a therapeutically effective amount of gemcitabine. GEMZAR^{®} (gemcitabine HCl) is 2'-deoxy-2',2'-difluorocytidine monohydrochloride (β-isomers), a nucleoside analog that exhibits antitumor activity. Gemcitabine may be used in monotherapy, or in combination with other agents (e.g., cisplatin, carboplatin, TAXOL® (paclitaxel)), to treat various cancers, including pancreatic cancer, breast cancer, non-small cell lung cancer, ovarian cancer, and bladder cancer. Gemcitabine exhibits cell phase specificity, primarily killing cells undergoing DNA synthesis (S-phase) and also blocking the progression of cells through the G1/S boundary. Without being limited by theory, it is believed that after a gemcitabine nucleotide is incorporated into DNA, only one additional nucleotide may be added to the growing DNA strands. Again not limited by theory, it is believed that DNA polymerase epsilon is unable to remove the gemcitabine nucleotide and repair the growing DNA strand (e.g., masked chain termination). In CEM T lymphoblastoid cells, gemcitabine induces internucleosomal DNA fragmentation, one of the characteristics of programmed cell death (e.g., apoptosis).

One skilled in the art may refer to general reference texts for detailed descriptions of known techniques discussed herein or equivalent techniques. These texts include Ausubel et al., Current Protocols in Molecular Biology, John Wiley and Sons, Inc. (2005); Sambrook et al., Molecular Cloning, A Laboratory Manual (3d ed.), Cold Spring Harbor Press, Cold Spring Harbor, New York (2000); Coligan et al., Current Protocols in Immunology, John Wiley & Sons, N.Y.; Enna et al., Current Protocols in Pharmacology, John Wiley & Sons, N.Y.; Fingl et al., The Pharmacological Basis of Therapeutics (1975), Remington's Pharmaceutical Sciences, Mack Publishing Co., Easton, PA, 18th edition (1990). These texts can, of course, also be referred to in making or using an aspect of the invention.

A compound of the present invention, or a pharmaceutically acceptable salt, prodrug, metabolite, analog or derivative thereof, can be incorporated into pharmaceutical compositions suitable for administration. Such compositions typically comprise the compound (i.e., including the active compound), and a pharmaceutically acceptable excipient or carrier. As used herein, "pharmaceutically acceptable excipient" or "pharmaceutically acceptable carrier" is intended to include any and all solvents, dispersion media, coatings, antibacterial and antifungal agents, isotonic and absorption delaying agents, and the like, compatible with pharmaceutical administration. Suitable carriers are described in the most recent edition of Remington's Pharmaceutical Sciences, a standard reference text in the field. Preferred examples of such carriers or diluents include, but are not limited to, water, saline, ringer's solutions, dextrose solution, and 5% human serum albumin. Pharmaceutically acceptable carriers include solid carriers such as lactose, terra alba, sucrose, talc, gelatin, agar, pectin, acacia, magnesium stearate, stearic acid and the like. Exemplary liquid carriers include syrup, peanut oil, olive oil, water and the like. Similarly, the carrier or diluent may include time-delay material known in the art, such as glyceryl monostearate or glyceryl distearate, alone or with a wax, ethylcellulose, hydroxypropylmethylcellulose, methylmethacrylate or the like. Other fillers, excipients, flavorants, and other additives such as are known in the art may also be included in a pharmaceutical composition according to this invention. Liposomes and non-aqueous vehicles such as fixed oils may also be used. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

The pharmaceutical compositions of this invention which are provided as part of the combination therapies may exist in the dosage form as a solid, semi-solid, or liquid such as, e.g., suspensions, aerosols or the like. Preferably the compositions are administered in unit dosage forms suitable for single administration of precise dosage amounts. The compositions may also include, depending on the formulation desired, pharmaceutically-acceptable, nontoxic carriers or diluents, which are defined as vehicles commonly used to formulate pharmaceutical compositions for animal or human administration. The diluent is selected so as not to affect the biological activity of the combination. Examples of such diluents are distilled water, physiological saline, Ringer's solution, dextrose solution, and Hank's solution. A preferred carrier for the solubilization of β-lapachone is hydroxypropyl beta cyclodextrin, a water solubilizing carrier molecule. Other water-solubilizing agents for combining with β-lapachone and/or an S-phase compound, such as Poloxamer, Povidone K17, Povidone K12, Tween 80, ethanol, Cremophor/ethanol, polyethylene glycol 400, propylene glycol and Trappsol, are contemplated. Furthermore, the invention is not limited to water-solubilizing agents, and oilbased solubilizing agents such as lipiodol and peanut oil, may also be used.

In addition, the pharmaceutical composition or formulation may also include other carriers, adjuvants, or nontoxic, nontherapeutic, nonimmunogenic stabilizers and the like. Effective amounts of such diluent or carrier will be those amounts which are effective to obtain a pharmaceutically acceptable formulation in terms of solubility of components, or biological activity, and the like. Liposome formulations, are also contemplated by the present invention, and have been described. See, e.g. U.S. Pat. No. 5,424,073.

For the purposes of the present invention, the G1/S phase drugs or compounds, or derivatives or analogs thereof, and the S phase drugs or compounds, or derivatives or analogs thereof, described herein include their pharmacologically acceptable salts, preferably sodium; analogs containing halogen substitutions, preferably chlorine or fluorine; analogs containing ammonium or substituted ammonium salts, preferably secondary or tertiary ammonium salts; analogs containing alkyl, alkenyl, aryl or their alkyl, alkenyl, aryl, halo, alkoxy, alkenyloxy substituted derivatives, preferably methyl, methoxy, ethoxy, or phenylacetate; and natural analogs such as naphthyl acetate. Further, the G1/S phase compounds or derivatives or analogs thereof, and the S phase compounds or derivatives or analogs thereof, described herein may be conjugated to a water-soluble polymer or may be derivatized with water-soluble chelating agents or radionuclides. Examples of water soluble polymers are, but not limited to: polyglutamic acid polymer, copolymers with polycaprolactone, polyglycolic acid, polyactic acid, polyacrylic acid, poly (2-hydroxyethyl 1-glutamine), carboxymethyl dextran, hyaluronic acid, human serum albumin, polyalginic acid or a combination thereof. Examples of water-soluble chelating agents are, but not limited to: DIPA (diethylenetriaminepentaacetic acid), EDTA, DTTP, DOTA or their water-soluble salts, etc. Examples of radionuclides include, but not limited to: ¹¹¹In, ⁹⁰Y, ¹⁶⁶Ho, ⁶⁸Ga, ^{99*m*}Tc, and the like.

Due to the water insolubility of β-lapachone, pharmaceutical carriers or solubilizing agents may be used to provide sufficient quantities of β-lapachone for use in the treatment methods of the present invention. See, *e.g.,* U.S. Patent Publication 20030091639 to Jiang et al.*,* and U.S. Patent Publication 20040001871 to Boothman et al. This publication describes the use of complexing agents such as cyclodextrins, including hydroxypropyl beta-cyclodextrin (HPBCD), to permit the solubilization of β-lapachone at levels sufficient for administration. See also U.S. Patent Publication 20040001871 to Boothman et al. In an embodiment, the G1/S phase drug, or an analog or derivative thereof, is administered with a pharmaceutically acceptable water solubilizing carrier molecule selected from the group consisting of Poloxamer, Povidone K17, Povidone K12, Tween 80, ethanol, Cremophor/ethanol, polyethylene glycol (PEG) 400, propylene glycol, Trappsol, alpha-cyclodextrin or derivatives or analogs thereof, beta-cyclodextrin or derivatives or analogs thereof, and gamma-cyclodextrin or derivatives or analogs thereof.

In one aspect, a compound of the present invention, or a pharmaceutically acceptable salt, prodrug, metabolite, analog or derivative thereof, is administered in a suitable dosage form prepared by combining a therapeutically effective amount (e.g., an efficacious level sufficient to achieve the desired therapeutic effect through inhibition of tumor growth, killing of tumor cells, treatment or prevention of cell proliferative disorders, etc.) of a compound of the present invention, or a pharmaceutically acceptable salt, prodrug, metabolite, analog or derivative thereof, (as an active ingredient) with standard pharmaceutical carriers or diluents according to conventional procedures (i.e., by producing a pharmaceutical composition of the invention). These procedures may involve mixing, granulating, and compressing or dissolving the ingredients as appropriate to attain the desired preparation.

A pharmaceutical composition of the invention is formulated to be compatible with its intended route of administration. Examples of routes of administration include parenteral, *e.g.,*, intravenous, intradermal, subcutaneous, oral (*e.g.*,, inhalation), transdermal (topical), and transmucosal administration. Although intravenous administration is preferred as discussed above, the invention is not intended to be limited in this respect, and the compounds can be administered by any means known in the art. Such modes include oral, rectal, nasal, topical (including buccal and sublingual) or parenteral (including subcutaneous, intramuscular, intravenous and intradermal) administration. For ease of administration and comfort to the patient, oral administration is generally preferred. However, oral administration may require the administration of a higher dose than intravenous administration. The skilled artisan can determine which form of administration is best in a particular case, balancing dose needed versus the number of times per month administration is necessary. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates, and agents for the adjustment of tonicity such as sodium chloride or dextrose. The pH can be adjusted with acids or bases, such as hydrochloric acid or sodium hydroxide. The parenteral preparation can be enclosed in ampoules, disposable syringes or multiple dose vials made of glass or plastic.

Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates, and agents for the adjustment of tonicity such as sodium chloride or dextrose. The pH can be adjusted with acids or bases, such as hydrochloric acid or sodium hydroxide. The parenteral preparation can be enclosed in ampoules, disposable syringes or multiple dose vials made of glass or plastic.

A compound or pharmaceutical composition of the invention can be administered to a subject in many of the well-known methods currently used for chemotherapeutic treatment. For example, for treatment of cancers, a compound of the invention may be injected directly into tumors, injected into the blood stream or body cavities or taken orally or applied through the skin with patches. The dose chosen should be sufficient to constitute effective treatment but not so high as to cause unacceptable side effects. The state of the disease condition (e.g., cancer, precancer, and the like) and the health of the patient should preferably be closely monitored during and for a reasonable period after treatment.

The term "therapeutically effective amount," as used herein, refers to an amount of a pharmaceutical agent to treat, ameliorate, or prevent an identified disease or condition, or to exhibit a detectable therapeutic or inhibitory effect. The effect can be detected by any assay method known in the art. The precise effective amount for a subject will depend upon the subject's body weight, size, and health; the nature and extent of the condition; and the therapeutic or combination of therapeutics selected for administration. Therapeutically effective amounts for a given situation can be determined by routine experimentation that is within the skill and judgment of the clinician. In a preferred aspect, the disease or condition to be treated is cancer. In another aspect, the disease or condition to be treated is a cell proliferative disorder.

For any compound, the therapeutically effective amount can be estimated initially either in cell culture assays, e.g., of neoplastic cells, or in animal models, usually rats, mice, rabbits, dogs, or pigs. The animal model may also be used to determine the appropriate concentration range and route of administration. Such information can then be used to determine useful doses and routes for administration in humans. Therapeutic/prophylactic efficacy and toxicity may be determined by standard pharmaceutical procedures in cell cultures or experimental animals, e.g., ED₅₀ (the dose therapeutically effective in 50% of the population) and LD₅₀ (the dose lethal to 50% of the population). The dose ratio between therapeutic and toxic effects is the therapeutic index, and it can be expressed as the ratio, ED₅₀/LD₅₀. Pharmaceutical compositions that exhibit large therapeutic indices are preferred. The dosage may vary within this range depending upon the dosage form employed, sensitivity of the patient, and the route of administration.

Dosage and administration are adjusted to provide sufficient levels of the active agent(s) or to maintain the desired effect. Factors which may be taken into account include the severity of the disease state, general health of the subject, age, weight, and gender of the subject, diet, time and frequency of administration, drug combination(s), reaction sensitivities, and tolerance/response to therapy. Long-acting pharmaceutical compositions may be administered every 3 to 4 days, every week, or once every two weeks depending on half-life and clearance rate of the particular formulation.

The pharmaceutical compositions containing active compounds of the present invention may be manufactured in a manner that is generally known, e.g., by means of conventional mixing, dissolving, granulating, dragee-making, levigating, emulsifying, encapsulating, entrapping, or lyophilizing processes. Pharmaceutical compositions may be formulated in a conventional manner using one or more pharmaceutically acceptable carriers comprising excipients and/or auxiliaries that facilitate processing of the active compounds into preparations that can be used pharmaceutically. Of course, the appropriate formulation is dependent upon the route of administration chosen.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, N.J.) or phosphate buffered saline (PBS). In all cases, the composition must be sterile and should be fluid to the extent that easy syringeability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, methods of preparation are vacuum drying and freeze-drying that yields a powder of the active ingredient plus any additional desired ingredient from a previously sterile-filtered solution thereof.

Oral compositions generally include an inert diluent or an edible pharmaceutically acceptable carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser, which contains a suitable propellant, e.g., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

In one aspect, the active compounds are prepared with pharmaceutically acceptable carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Pat. No. 4,522,811.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved.

In therapeutic applications, the dosages of the pharmaceutical compositions used in accordance with the invention vary depending on the agent, the age, weight, and clinical condition of the recipient patient, and the experience and judgment of the clinician or practitioner administering the therapy, among other factors affecting the selected dosage. Generally, the dose should be sufficient to result in slowing, and preferably regressing, the growth of the tumors and also preferably causing complete regression of the cancer. Dosages can range from about 0.01 mg/kg per day to about 3000 mg/kg per day. In preferred aspects, dosages can range from about 1 mg/kg per day to about 1000 mg/kg per day. In an aspect, the dose will be in the range of about 0.1 mg/day to about 70 g/day; about 0.1 mg/day to about 25 g/day; about 0.1 mg/day to about 10 g/day; about 0.1 mg to about 3g/day; or about 0.1 mg to about 1 g/day, in single, divided, or continuous doses (which dose may be adjusted for the patient's weight in kg, body surface area in m², and age in years). An effective amount of a pharmaceutical agent is that which provides an objectively identifiable improvement as noted by the clinician or other qualified observer. For example, regression of a tumor in a patient may be measured with reference to the diameter of a tumor. Decrease in the diameter of a tumor indicates regression. Regression is also indicated by failure of tumors to reoccur after treatment has stopped. As used herein, the term "dosage effective manner" refers to amount of an active compound to produce the desired biological effect in a subject or cell.

A compound of the present invention may be administered in combination with an S phase compound, such as an gemcitabine, in any manner found appropriate by a clinician in generally accepted efficacious dose ranges, such as those described in the Physician's Desk Reference, 59th Edition, Thomson PDR (2005)("PDR"). In general, gemcitabine is administered intravenously at dosages from about 10 mg/m² to about 10,000 mg/m², preferably from about 100 mg/m²to about 2000 mg/m², and most preferably about 500 to about 1500 mg/m². In an embodiment, gemcitabine is administered intravenously at a dosage from approximately 100 mg/m² to about 2000 mg/m². In an embodiment, gemcitabine is administered intravenously at a dosage of approximately 1000 mg/m². Dosage can be repeated, e.g., once weekly, preferably for about 1 to 6 weeks. It is preferred that dosages be administered over a time period of about 30 minutes to about 6 hours, and typically over a period of about 3 hours.

The S phase drug, such as an gemcitabine, will be administered in a similar regimen with a G1/S phase drug, such as β-lapachone or an analog or derivative thereof, although the amounts will preferably be reduced from that normally administered. It is preferred, for example, that the gemcitabine be administered at the same time or after the β-lapachone has administered to the patient. When the gemcitabine is administered after the β-lapachone, the gemcitabine is advantageously administered about 24 hours after the β-lapachone has been administered.

The combination therapy agents described herein may be administered singly and sequentially, or in a cocktail or combination containing both agents or one of the agents with other therapeutic agents, including but not limited to, immunosuppressive agents, potentiators and side-effect relieving agents. As aforesaid, the therapeutic combination, if administered sequentially, may be more effective when the G1/S phase drug component (e.g., β-lapachone) is administered prior to the S phase drug, e.g., gemcitabine. For example, a dose of the G1/S phase drug component (e.g., β-lapachone) is administered at least one hour (more preferably at least 2 hours, 4 hours, 8 hours, 12 hours, or 24 hours) prior to administration of a dose of the S phase drug, e.g., gemcitabine. In another embodiment, a dose of the G1/S phase drug component (*e.g.,* β-lapachone) is administered at least one hour (more preferably at least 2 hours, 4 hours, 8 hours, 12 hours, or 24 hours) following administration of a dose of the S phase drug, e.g., gemcitabine. The therapeutic agents will preferably be administered intravenously or otherwise systemically by injection intramuscularly, subcutaneously, intrathecally or intraperitoneally. In an embodiment, the S phase drug is administered simultaneously with or following administration of the G1/S phase drug. In another embodiment, the S phase drug is administered following administration of the G1/S phase drug. In another embodiment, the S drug is administered within 24 hours after the G1/S phase drug is administered.

The other component of the combination therapy for combination with the S phase drug or compound is the G1/S phase drug, which is preferably β-lapachone or an analog or derivative thereof.

β-lapachone has been shown to have a variety of pharmacological effects. β-lapachone has been shown to be a DNA repair inhibitor which sensitizes cells to DNA damaging agents (Boorstein, R.J., et al., (1984) Biochem. Biophys. Res. Commun., 118:828-834; Boothman, D.A., et al., (1989) J. Cancer Res., 49:605-612). β-lapachone is generally well-tolerated in dogs, rats, and mice.

The present invention provides a method of treating cancer or a precancerous condition or preventing cancer in a subject, the method comprising administering to the subject a therapeutically effective amount of a pharmaceutical composition comprising β-lapachone, or a derivative or analog thereof, or pharmaceutically acceptable salt thereof, or a metabolite thereof, and a pharmaceutically acceptable carrier such that the composition maintains a plasma concentration of about 0.15 µM to about 50 µM and treats the cancer or precancerous condition or prevents the cancer. In one aspect, the plasma concentration can be about 0.1 µM to about 100 µM, about 0.125 µM to about 75 µM; about 0.15 µM to about 50 µM; about 0.175 µM to about 30 µM; and about 0.2 µM to about 20 µM. In another aspect, the pharmaceutical composition can maintain a suitable plasma concentration for at least a month, at least a week, at least 24 hours, at least 12 hrs, at least 6 hrs, at least 1 hour. In a further aspect, a suitable plasma concentration of the pharmaceutical composition can be maintained indefinitely. In yet another aspect, the subject can be exposed to the pharmaceutical composition in a AUC (area under the curve) range of about 0.5 µM-hr to about 100 µM-hr, about 0.5 µM-hr to about 50 µM-hr, about 1 µM-hr to about 25 µM-hr, about 1 µM-hr to about 10 µM-hr; about 1.25 µM-hr to about 6.75 µM-hr, about 1.5 µM-hr to about 6.5 µM-hr. The pharmaceutical composition can be administered at a dosage from about 2 mg/m² to 5000 mg/m² per day, more preferably from about 20 mg/m² to 2000 mg/m² per day, more preferably from about 20 mg/m² to 500 mg/m² per day, most preferably from about 30 to 300 mg/m² per day. Preferably, 2 mg/m² to 5000 mg/m² per day is the administered dosage for a human. In another aspect, the pharmaceutical composition can be administered at a dosage from about 10 to 1,000,000 µg per kilogram body weight of recipient per day; preferably about 100 to 500,000 µg per kilogram body weight of recipient per day, more preferably from about 1000 to 250,000 µg per kilogram body weight of recipient per day, most preferably from about 10,000 to 150,000 µg per kilogram body weight of recipient per day. One of ordinary skill in the art can determine the appropriate dosage amount in mg/m² per day or µg per kilogram body weight of recipient per day depending on subject to which the pharmaceutical composition is to be administered.

As with the use of other chemotherapeutic drugs, the individual patient will be monitored in a manner deemed appropriate by the treating physician. Dosages can also be reduced if severe neutropenia or severe peripheral neuropathy occurs, or if a grade 2 or higher level of mucositis is observed, using the Common Toxicity Criteria of the National Cancer Institute.

In administering a G1/S phase compound such as β-lapachone, the normal dose of such compound individually is utilized as set forth above. However, when combination therapies are used, it is preferable to use a lower dosage -- preferably 75% or less of the individual amount, more preferably 50% or less, still more preferably 40% or less. The term "effective amount," as used herein, refers to an amount effective to treat the disease condition in combination with any other active agent in a combination regimen according to the invention.

In therapeutic applications, the dosages of the agents used in accordance with the invention vary depending on the agent, the age, weight, and clinical condition of the recipient patient, and the experience and judgment of the clinician or practitioner administering the therapy, among other factors affecting the selected dosage. Generally, the dose should be sufficient to result in slowing, and preferably regressing, the growth of the tumors and also preferably causing complete regression of the cancer. An effective amount of a pharmaceutical agent is that which provides an objectively identifiable improvement as noted by the clinician or other qualified observer. Regression of a tumor in a patient is typically measured with reference to the diameter of a tumor. Decrease in the diameter of a tumor indicates regression. Regression is also indicated by failure of tumors to reoccur after treatment has stopped. In preferred embodiments, a decrease in tumor size or burden of at least 20%, more preferably 50%, 80%, 90%, 95% or 99% is preferred.

The pharmaceutical compositions can be included in a container, pack, or dispenser together with instructions for administration.

### EXAMPLES

### Example 1. β-lapachone induces cell death of the G480 lung cancer cell line in vitro.

Exponentially growing cells are seeded at 1000 per well in six-well plates and allowed to attach for 48 hours. Drugs are added to dishes in less than 5 µl of concentrated solution (corresponding to a final DMSO concentration of less that 0.1%). β-lapachone is dissolved at a concentration of 20 mM in DMSO and diluted in complete media. Control plates receive the same volume of DMSO alone. After 1-4 hours exposure, cells are rinsed and drug-free medium is added. Cultures are left undisturbed for 10-20 days to allow for colony formation and then are fixed and stained with modified Wright-Giemsa stain (Sigma). Colonies of greater than 30 cells are scored as survivors. Cells are maintained at 37 °C in 5% CO₂ in complete humidity.

Treatment of G480 lung cancer cells for 4 hours with β-lapachone at 4 µM resulted in 68% cell death (*i.e.*, survival of 32% of cells) in comparison to treatment with carrier alone. Figure 1. Table 1 below provides a summary of the results. The number of colonies in control well was taken as 100% survival. Treated wells are presented as percentage of control. Data are given as average (+SEM) from three independent experiments.

**Table 1**

| | | **Colonies (percent control)** | | |
|---|---|---|---|---|
| **Tissue Origin** | **Cell Line** | **B-lapachone** | **Taxol** | **B-lapachone + Taxol** |
| Lung | G480 | 32 (0.3) | 39(2.6) | 2 (0.1) |

### Example 2. β-lapachone induces cell death of the A549 lung cancer cell line in vitro.

Exponentially growing cells are seeded at 250, 1000, or 5000 cells per well (2.5 ml) in six-well plates and allowed to attach for 24 hours. β-lapachone is dissolved at a concentration of 20 mM in DMSO and diluted in complete media. β-lapachone (0.5 ml) is added at 6-fold the final concentration to a total volume of 3.0 ml/well. Control plates receive the same volume of DMSO alone. After a 4 hour exposure the drug is carefully removed, and drug-free medium is added. Cultures are left undisturbed for 14-21 days to allow for colony formation and then are fixed and stained with crystal violet stain (Sigma). Colonies of greater than 50 cells are scored as survivors. Cells are maintained at 37 °C in 5% CO₂ in complete humidity. The results of three replicates are provided in the Table 2 below. Each "Replicate Result" represents the result of a separate experiment.

**Table 2**

| **Tissue Origin** | **Cell Line** | **LC₅₀(µM) (Replicate Results)** |
|---|---|---|
| Lung | A549 | 2.07 |
| Lung | A549 | 1.81 |
| Lung | A549 | 1.79 |

### Example 3. β-lapachone induces cell death in lung cancer cell lines in the NCI60 in vitro screen.

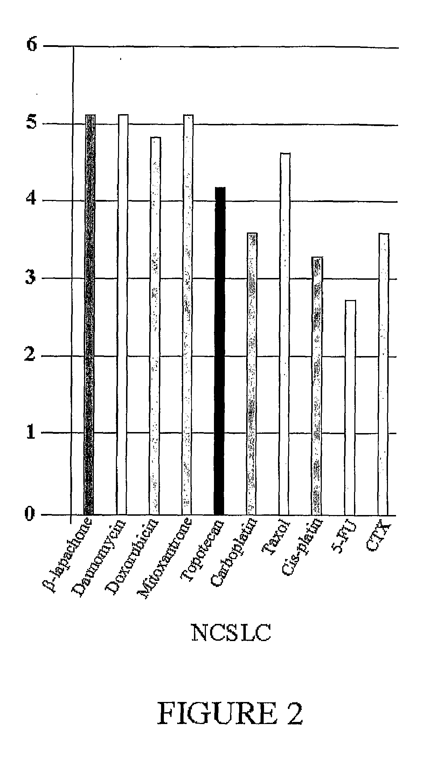

β-lapachone is tested in the NCI in vitro screen of 60 cancer cell lines, which allows comparison with other anti-tumor agents under standardized conditions. The NCI assays are performed under standardized conditions not designed to mimic the conditions of dosing and use the sulforhodamine B assay as the endpoint. The NCI set of 60 lines includes nine non-small cell lung cancer cell lines (A549/ATCC, EKVX, HOP-62, HOP-92, NCI-H226, NCI-H23, NCI-H322M, NCI-H460, NCI-H522). β-lapachone is broadly active against many cell types, with LC₅₀ (log10 molar concentration causing 50% lethality) between -4.5 and -5.3, and mean of - 5.07 across all cells. When compared to many FDA approved chemotherapeutic agents for common cancer types with publicly available data, none of the compared approved drugs exceed the mean of β-lapachone across all cells and only mitoxantrone equals it. Figure 2.

### Example 4. β-lapachone does not select for drug-resistant lung cancer cell populations in vitro.

Exponentially growing A549 lung cancer cells are plated at 2 x 10⁵ cells in 60-mm dishes and allowed to attach for 48 hours. β-lapachone is dissolved at a concentration of 20 mM in DMSO and diluted in complete media. Growth media is removed from the cultures and β-lapachone is added at final drug concentrations of 1, 2, 5, 10 and 20 µM. After a 4 hour exposure, the drug media is aspirated and the cultures are washed with PBS, trypsinized, and plated at 200-40,000 cells/100-mm dish. Variable cell numbers are plated to yield approximately 50-200 colonies/drug concentration. Cultures are left undisturbed for 14-21 days to allow for colony formation and are fixed and stained with crystal violet stain. Colonies of greater than 50 cells are scored as survivors. For each cell line, two colonies ("Clone A" and "Clone B") are selected from those surviving >LC₉₉ concentrations of β-lapachone were isolated, expanded and used to repeat the assay. Individual cancer cells surviving 4-h exposures to >LC₉₉ concentrations of β-lapachone were isolated and cultured, then retested for sensitivity to β-lapachone.

As shown in Table 3 below, exponentially growing cultures of surviving lung cancer cells show the same LC₅₀ concentrations as the initial cultures. Attempts to generate resistance by long term continuous exposure of tumor cell lines to sublethal concentrations of β-lapachone has thus far also been unsuccessful. *In vitro* studies therefore suggest that β-lapachone does not select for resistant cell populations.

**Table 3**

| **Tissue Origin** | **Cell Line** | **LC₅₀, µM** |
|---|---|---|
| Lung | A549 WT | 1.79 |
| | Clone A | 1.69 |
| | Clone B | 1.50 |

### Example 5. β-lapachone potently reduces mean tumor volume in a human lung cancer xenograft mouse model.

The anti-tumor activity of β-lapachone is examined using a human lung cancer xenograft model. Athymic female nude mice (Ncr) are inoculated subcutaneously with 4x10⁶ A549 human lung cancer cells, and the tumors are allowed to grow to 50 mm³ in size. The animals are randomized into three groups of seven animals per group. Animals are treated intraperitoneally every three days with either β-lapachone (40 mg/kg or 60 mg/kg) or vehicle control, for a total of 8 treatments per animal. Mean tumor volume is then analyzed.

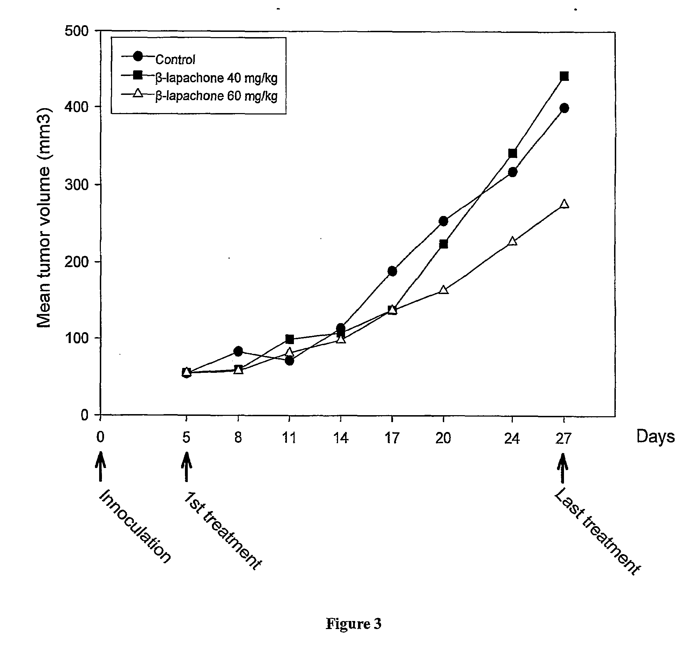

Treatment with β-lapachone at 60 mg/kg reduced the mean tumor volume of xenografted human lung cancer by approximately 50%. (Fig. 3) No sign of significant toxicity was noted for any of the treatment regimens. *In vitro* experiments using cell lines of various tissue origins corroborate that β-lapachone is relatively non-toxic to normal cells.

### Example 6. β-lapachone administered in monotherapy, or in combination with gemcitabine (GEMZAR^{®}), potently reduces mean tumor volume in a human lung cancer xenograft mouse model.

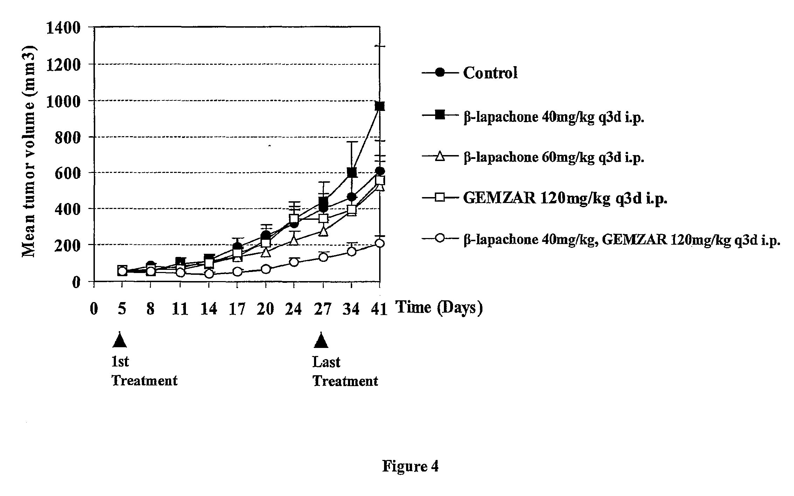

The anti-tumor activity of β-lapachone is examined using a human lung cancer xenograft model. Briefly, athymic female nude mice (Ncr) are inoculated subcutaneously with 4x10⁶ A549 human lung cancer cells, and the tumors are allowed to grow to approximately 50mm³ in size. The animals are randomized into five groups of seven animals per group, and treated intraperitoneally every three days with one of the following five regimens: β-lapachone at 40mg/kg in 40% hydroxypropy-β-cyclodextran ("HPBCD"); β-lapachone at 60mg/kg in 40% HPBCD; gemcitabine (GEMZAR^{®}) at 120mg/kg in PBS; β-lapachone (40mg/kg) + gemcitabine (120mg/kg); or vehicle control (40% HPBCD). The combination therapy group receives treatments with β-lapachone and gemcitabine at the indicated concentrations on the same day, every three days. Mice receive a total of eight treatments. Mean tumor volume is analyzed; data points in Figure 4 represent the arithmetic mean +/- SEM of five tumors.

As shown in Figure 4, treatment with either β-lapachone (60mg/kg) or gemcitabine (120mg/kg) alone retarded tumor growth to a similar extent during treatment. *See, e.g.,* Figure 4, days 24 and 27 of treatment. Animals treated with β-lapachone (40mg/kg) in combination with gemcitabine (120mg/kg) showed an unexpected synergistic retardation of tumor growth. β-lapachone dosed at 60 mg/kg was shown to be more effective at retarding tumor growth than ARQ β-lapachone dosed at 40 mg/kg. No significant toxicity was noted for any of the treatment regimens. We conclude from this study that β-lapachone either alone, or in combination with gemcitabine, can be safely dosed in regimens that are effective for treating lung cancer.

## Claims

1. A composition comprising β-lapachone, or a pharmaceutically acceptable salt thereof, in combination with a pharmaceutically acceptable carrier, for use in the treatment or prevention of a cell proliferative disorder of the lung.

2. A composition as claimed in claim 1, wherein said cell proliferative disorder is lung cancer.

3. A composition according to claim 1, wherein said cell proliferative disorder is a precancerous condition of the lung.

4. A composition according to claim 1, wherein said cell proliferative disorder is hyperplasia or metaplasia of the lung.

5. A composition according to claim 2, wherein said lung cancer is metastatic lung cancer.

6. A composition according to claim 2, wherein said lung cancer is small cell lung cancer.

7. A composition according to claim 2, wherein said lung cancer is non-small cell lung cancer.

8. A composition according to claim 1, wherein said β-lapachone, or a pharmaceutically acceptable salt thereof, is for parenteral administration.

9. A composition according to claim 1, wherein said β-lapachone, or a pharmaceutically acceptable salt thereof, is for intravenous administration.

10. A composition according to claim 1, wherein said β-lapachone, or a pharmaceutically acceptable salt thereof, is for oral administration.

11. A composition according to claim 1, wherein said β-lapachone, or a pharmaceutically acceptable salt thereof, is for topical administration.
